# EUROPÄISCHE PATENTSCHRIFT

(11) **EP 0 556 660 B1**
(45) Veröffentlichungstag und Bekanntmachung des Hinweises auf die Patenterteilung: **28.05.1997**
(21) Anmeldenummer: 93101765.1
(22) Anmeldetag: 05.02.1993
(51) Int. Cl.: C07C 59/01, A61K 7/48

(54) **Verwendungen von alpha-Hydroxyfettsäuren**
Utilisation of fatty alpha-hydroxy acids
Utilisation d'acides gras alpha-hydroxyliques

(30) Priorität: 13.02.1992 DE 4204321
(43) Veröffentlichungstag der Anmeldung: 25.08.1993
(73) Patentinhaber: Beiersdorf Aktiengesellschaft, D-20245 Hamburg (DE)
(72) Erfinder: Schmucker, Robert, Dr., W-2000 Hamburg 61 (DE); Paal, Michael, Dr. Dr., W-2000 Hamburg 63 (DE); Sauermann, Gerhard, Dr., W-2351 Wiemersdorf (DE); Schreiner, Volker, Dr., W-2000 Hamburg 20 (DE); Traupe, Bernd, W-2000 Hamburg 61 (DE); Eigener, Ulrich, Dr., W-2000 Hamburg 65 (DE)

(56) Entgegenhaltungen:
- EP-A- 0 261 812
- EP-A- 0 347 198
- EP-A- 0 413 528
- EP-A- 0 435 483
- EP-A- 0 442 708

## Beschreibung

Die Erfindung betrifft Verwendungen von alpha-Hydroxyfettsäuren und ihren Salzen.

Gegenstand der Erfindung sind die Verwendung von alpha-Hydroxyfettsäuren als desodorierende Wirkstoffe und die Verwendung von alpha-Hydroxyfettsäuren als Konservierungshelfer, insbesondere in kosmetischen Zubereitungen, sowie auch die Verwendung von alpha-Hydroxyfettsäuren zum Binden und Komplexieren von Ionen, insbesondere diese Verwendung in kosmetischen Zubereitungen, z.B. als Ersatz für EDTA.

### Desodorierendes kosmetisches Mittel

Schweiß erscheint an der Hautoberfläche als geruchloses, steriles Sekret. Erst durch die Verstoffwechselung bestimmter Schweißinhaltsstoffe durch die Bakterien der Achselflora entstehen Substanzen, die für den Achselgeruch verantwortlich sind. Folglich kann man durch den Einsatz von antimikrobiell wirksamen Substanzen in Deodorantien einen desodorierenden Effekt erzielen.

Es ist bekannt, daß z.B. Laurinsäure oder deren Derivate wie z.B. Glycerin-mono-laurat (GML) eine antimikrobielle Wirksamkeit gegenüber grampositiven Bakterien besitzen.

Darüberhinaus existieren in der Literatur Hinweise bezüglich der antimikrobiellen Wirksamkeit von alpha-Hydroxyfettsäureseifen (A. H. Eggerth (1929), "The Germicidal Action of Hydroxy Soaps", Jour. Exp. Med. 50, 299-313).

So wurden verschiedene Bakterienspezies auf ihre Sensitivität gegenüber alpha-Hydroxyfettsäuren getestet. Es ist heute bekannt, daß die Corynebakterien für den Achselgeruch verantwortlich sind. Da dieser Organismentyp jedoch nicht beschrieben wurde, gibt es keinen Hinweis auf eine Deowirksamkeit der alpha-Hydroxyfettsäuren. Auch ein Analogieschluß von den sensitiven Bakterien auf Corynebakterien ist nicht möglich, da festgestellt wurde, daß die antimikrobielle Wirksamkeit bei verschiedenen Testorganismen stark variierte. Schließlich ist dem Fachmann bekannt, daß eine in vitro festgestellte antimikrobielle Wirksamkeit keinen sicheren Hinweis bezüglich einer Deowirksamkeit erlaubt.

Weiterhin sind aus den deutschen Offenlegungsschriften DE-A-31 47 777 und DE-A-30 20 304 bereits desodorierende kosmetische Mittel mit einem Gehalt an Wollwachssäuren oder deren Alkali- oder Ammoniumsalzen bekannt. Diese haben sich jedoch nicht immer als zufriedenstellend erwiesen.

Aus der DE-A-31 47 777 ist weiter bekannt, daß der in den Wollwachssäuren vorhandene Gehalt an nicht hydroxylierten Iso-C₁₄- und Iso-C₁₆-Carbonsäuren mit bekannter bakterizider Wirkung bewirkt, daß Stifte eine gute desodorierende Wirkung aufweisen, ohne des Zusatzes eines speziellen Deowirkstoffes zu bedürfen. In den verwendeten Wollwachssäuregemischen können zwar auch Hydroxycarbonsäuren enthalten sein, aber es findet sich kein Hinweis auf eine Wirksamkeit und Verwendungsmöglichkeit als desodorierender Stoff.

Aufgabe der Erfindung ist es, zur Verwendung als desodorierend wirkende Stoffe geeignete Wirkstoffe bereitzustellen und desodorierende kosmetische Mittel zu schaffen, die die Nachteile des Standes der Technik nicht aufweisen und insbesondere einen im Hinblick auf die Wirksamkeit und Hautschonung verbesserten desodorierenden Wirkstoff Zur Verfügung zu stellen sowie kosmetische Zubereitungen, die diesen Wirkstoff enthalten.

Diese Aufgabe wird gelöst durch die Verwendung von alpha-Hydroxyfettsäuren mit 12 bis 24 Kohlenstoffatomen oder deren Salzen als desodorierende Wirkstoffe in kosmetischen Zubereitungen, wobei die alpha-Hydroxyfettsäuren sowohl einzeln als auch im Gemisch vorliegen können.

Bevorzugt werden kosmetisch verträgliche Salze, insbesondere Alkali oder Ammoniumsalze, beispielsweise Natriumsalze oder Kaliumsalze.

Gemische können von zwei, mehreren aber auch einer größeren Anzahl von Verbindungen gebildet werden und diese Verbindungen in unterschiedlichen Gewichtsmengen enthalten. So können aus natürlichen Quellen stammende Fraktionen eine größere Zahl aufgrund von Homologen oder Strukturisomeren aufweisen, synthetisierte Säuren können dagegen aus jeweils einer Verbindung bestehen. Erfindungsgemäß soll mindestens eine der genannten alpha-Hydroxysäuren verwendet werden oder in der Zubereitung enthalten sein.

Die erfindungsgemäßen desodorierenden alpha-Hydroxyfettsäuren können geradkettig oder verzweigt sein. Bevorzugt werden die gesättigten aliphatischen alpha-Hydroxyfettsäuren.

Es wurde überraschenderweise gefunden, daß die erfindungsgemäßen alpha-Hydroxyfettsäuren und ihre Salze sowie die damit erhältlichen kosmetischen Mittel eine ausgeprägte desodorierende Wirkung besitzen.

Durch die Hydroxygruppe enthalten die erfindungsgemäßen Fettsäuren mindestens ein asymmetrisches Kohlenstoffatom, und sie können als Racemat, Diastereoisomere oder in der optisch aktiven D- oder L-Form vorliegen. Bevorzugt werden entweder die Verwendung der D-Form oder die der L-Form sowie Zubereitungen, die entweder die D-Form oder die L-Form enthalten.

Bevorzugte erfindungsgemäße desodorierende alpha-Hydroxyfettsäuren oder deren Salze enthalten 14 bis 20 Kohlenstoffatome, insbesondere aber 16 bis 18 Kohlenstoffatome. Vorteilhaft sind auch Gemische der in solchen Bereichen liegenden alpha-Hydroxyfettsäuren.

Besonders bevorzugt sind die folgenden alpha-Hydroxyfettsäuren und ihre Salze:
alpha-Hydroxy-hexadecansäure
   (alpha-Hydroxy-palmitinsäure)
alpha-Hydroxy-tetradecansäure
alpha-Hydroxy-stearinsäure
   oder
alpha-Hydroxy-16-methylheptadecansäure.

Ganz besonders bevorzugt werden die optischen Isomere der vorstehend genannten Hydroxyfettsäuren und Gemische, d.h. jeweils der D- oder L-Form, insbesondere aber der D-Form.

Erfindungsgemäß können vorteilhaft aus Wollwachs gewonnenene alpha-Hydroxyfettsäuren eingesetzt werden, z.B. auch gemäß folgendem Isolierungsverfahren erhältliche alpha-Hydroxy-Fettsäuregemische, die aus Wollwachssäuregemischen gewonnen werden. Sie fallen vorzugsweise als D-Enantiomeren-Gemisch an. Auch die nach bekannten Verfahren daraus erhältlichen Einzelverbindungen sind bevorzugt.

Es ist möglich, daß beispielsweise aus Wollwachssäuregemischem gewonnene erfindungsgemäße alpha-Hydroxyfettsäuren und deren Gemische Anteile an (nicht hydroxylierten) Fettsäuren enthalten.

Bevorzugt werden solche Mischungen, in denen dieser Fettsäureanteil nicht zur Deowirkung beiträgt und besonders bevorzugt werden solche Mischungen, in denen ein möglicher Anteil an iso-Fettsäuren, insbesondere an iso-C₁₄ und iso-C₁₆-Carbonsäuren, nicht zur Deowirkung beiträgt. Solche Fettsäuren besitzen keine oder nur sehr geringe desodorierende Eigenschaften.

Besonders bevorzugt werden solche Mischungen, in denen der Gewichtsanteil der alpha-Hydroxyfettsäuren bezogen auf das Gesamtgewicht der Mischungen höher ist als in den bekannten Wollwachssäuregemischen. Solche Fraktionen, in denen der alpha-Hydroxyfettsäure-Anteil angereichert ist, sind beispielsweise nach den folgenden Isolierungs- und Reinigungsverfahren erhältlich.

Die erfindungsgemäßen desodorierenden alpha-Hydroxyfettsäuren sind bekannt oder nach bekannten Verfahren erhältlich. In der Synthese anfallende Racemate lassen sich in üblichen Verfahren in die Isomere aufspalten.

Geeignete alpha-Hydroxyfettsäuren können auch durch ein Verfahren zur Trennung und Isolierung von Hydroxyfettsäuren und Fettsäuren aus Wollwachssäuregemischen erhalten werden, das dadurch gekennzeichnet ist, daß man entweder
a) in einem ersten Schritt ein Wollwachssäuregemisch mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht, oder in umgekehrter Reihenfolge
b) in einem ersten Schritt ein Wollwachssäuregemisch mit mindestens einem unpolaren Lösungsmittel behandelt, wobei sich eine Dispersion oder Suspension bildet, den festen Anteil, der die Hydroxyfettsäuren enthält, davon abtrennt und die verbleibende flüssige Phase vom Lösungsmittel befreit, wobei ein Rückstand entsteht der vorwiegend aus nicht hydroxylierten, langkettigen höheren Fettsäuren besteht, und gegebenenfalls in einem zweiten Schritt den verbliebenen festen Anteil mit den Hydroxyfettsäuren mit mindestens einem polaren Lösungsmittel behandelt, wobei sich ein Teil des festen Anteiles löst, die erhaltene Lösung von ungelösten Teilen, die vorwiegend aus nicht hydroxylierten höheren Fettsäuren kurzer Kettenlänge bestehen, befreit und von der Lösung das Lösungsmittel entfernt, wodurch man einen Rückstand erhält, der die Hydroxyfettsäuren enthält, und
c) gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang, vor den Schritten a) oder b), in entsprechender Weise mit dem Wollwachssäuregemisch vornimmt.

Bevorzugt wird die unter a) beschriebene Reihenfolge der Einzelschritte. Das natürliche Wollwachssäuregemisch (oder auch Wollwachssäuren-Gemisch) wird also zuerst mit dem polaren Lösungsmittel und dann mit dem unpolaren Lösungsmittel wie angegeben behandelt.

Auch die in den Verfahrensteilen a) und b) jeweils im ersten Schritt isolierten Gemische enthalten alpha-Hydroxyfettsäuren in höheren Gewichtsanteilen als in den Ausgangsgemischen und können z.B. in kosmetischen Zubereitungen verwendet werden. Vorzugsweise folgen aber die jeweiligen fakultativen genannten zweiten Schritte, die zu einer weiteren Anreicherung der alpha-Hydroxyfettsäuren führen.

Vorzugsweise schließt sich an die Trennungs- und Isolierungsschritte des Verfahrens der fakultative Reinigungsschritt an. Dies kann dann besonders zweckmäßig sein, wenn die erhaltenen Hydroxyfettsäuren und Fettsäuren in kosmetischen Zubereitungen verwendet werden. Auf diese Weise können noch aus dem Wollwachs oder der Wollwachsspaltung herrührende Verunreinigungen entfernt werden. Es ist aber auch möglich, diesen Reinigungsschritt vor Beginn der Isolierungsschritte a) und b) vorzunehmen, also direkt das Wollwachssäuregemisch, das als Ausgangsprodukt dient, einzusetzen.

Das Verfahren bezieht sich vorzugsweise auf alphabeziehungsweise 2-Hydroxyfettsäuren.

Bevorzugte polare Lösungsmittel sind Alkohole und Ketone, vorzugsweise Methanol, Ethanol, n-Propanol, iso-Propanol, Aceton oder Ethylmethylketon, insbesondere Ethanol oder Methanol, sowie Gemische dieser Lösungsmittel, insbesondere mit Methanol oder Ethanol.

Bevorzugte unpolare Lösungsmittel sind aliphatische und cyclische Kohlenwasserstoffe oder alkylierte Aromaten wie Alkylbenzole, vorzugsweise Pentan, Hexan, Heptan und Oktan oder die Isomere dieser Lösungsmittel, Cyclohexan, Toluol oder Xylol, insbesondere aber Heptan oder Cyclohexan, sowie Gemische dieser Lösungsmittel, insbesondere mit Cyclohexan oder Heptan.

Bevorzugt können die erfindungsgemäßen Verfahrensschritte in folgender Weise in an sich bekannter Weise allgemein wie folgt ausgeführt werden:

Das Behandeln eines Säuregemisches oder einer Säurekomponente mit einem Lösungsmittel erfolgt, indem das Lösungsmitel zugegeben und die Stoffe vermischt werden und insbesondere in intensiven, ausreichenden Kontakt gebracht werden, beispielsweise durch Rühren oder Schütteln, vorzugsweise Rühren. Dabei kann sich überwiegend eine Lösung oder eine Suspension oder Dispersion ergeben.

Von diesen Gemischen können feste, ungelöste Anteile abgetrennt werden oder die Lösung kann von ungelösten Anteilen befreit werden. Dies kann z.B. durch Abpressen, Filtrieren oder Zentrifugieren geschehen, insbesondere durch Filtrieren.

Lösungen oder flüssige Phasen können von Lösungsmitteln befreit werden oder das Lösungsmittel kann aus der Lösung entfernt werden. Hierzu wird das Lösungsmittel vorzugsweise unter Erwärmung und/oder vermindertem Druck abgezogen oder abdestilliert.

Das Verfahren wird vorzugsweise wie folgt durchgeführt:
a) Das als Ausgangsprodukt dienende Wollwachssäuregemisch wird mit dem polaren Lösungsmittel vermischt und in intensiven Kontakt gebracht, insbesondere gerührt oder geschüttelt. Hierzu können übliche Rührer und übliche Mischwerke oder Rührwerke verwendet werden. Die Temperatur beträgt dabei vorzugsweise 0°C bis 30°C, insbesondere etwa 20°C.
   Die Lösungsmittel-Gewichtsmenge beträgt vorzugsweise das 1 bis 20-fache, insbesondere 2 bis 10-fache der Wollwachssäuregemisch-Gewichtsmenge. Die Behandlungszeit kann vorzugsweise 0,5 bis 20 Stunden, insbesondere 1 bis 10 Stunden betragen. Man erhält nach Filtration als Rückstand etwa 10 bis 20 Gewichtsprozent des Ausgangsgewichtes nicht hydroxylierte, langkettige höhere Fettsäuren, die insbesondere etwa 18 bis 32 Kohlenstoffatome besitzen.
   Die verbliebene Lösung wird unter Erwärmung bei vermindertem Druck vom Lösungsmittel befreit und es ergibt sich ein Rückstand von etwa 80 bis 90 Gewichtsprozent des Ausgangsgewichtes. Dieser Rückstand wird dann gegebenenfalls mit dem unpolaren Lösungsmittel vermischt und in intensiven Kontakt gebracht, insbesondere gerührt oder geschüttelt. Hierzu können übliche Rührer und Mischwerke oder Rührwerke verwendet werden. Die Temperatur beträgt dabei vorzugsweise 0 bis 30°C, insbesondere etwa 20°C.
   Die Lösungsmittel-Gewichtsmenge beträgt vorzugsweise das 1 bis 20-fache, insbesondere 2 bis 10-fache der Gewichtsmenge des Rückstandes. Die Behandlungszeit kann vorzugsweise 1 bis 40 Stunden, insbesondere 2 bis 20 Stunden betragen. Die sich bildende Lösung wird vom Rückstand z.B. durch erneute Filtration getrennt und der Rückstand kann zweckmäßigerweise einmal oder zweimal mit dem unpolaren Lösungsmittel gewaschen werden. Die Menge beträgt dann etwa 10 Gewichtsprozent der für die Behandlung gewählten Lösungsmittelmenge. Der Rückstand enthält die Hydroxyfettsäuren in einer Menge von etwa 20 Gewichtsprozent, bezogen auf die Ausgangsmenge Wollwachssäuregemisch.
   Die verbleibende Lösung ergibt nach Entfernen des Lösungsmittels bei vermindertem Druck etwa eine Menge von 60 bis 70 Gewichtsprozent des Ausgangsgewichtes eines wachsartigen Rückstandes, der vorwiegend aus nicht hydroxylierten, kurzkettigen höheren Fettsäuren besteht, die insbesondere etwa 14 bis 16 Kohlenstoffatome besitzen.
b) Geht man in umgekehrter Reihenfolge vor, wird das als Ausgangsprodukt dienende Wollwachssäuregemisch zunächst mit dem unpolaren Lösungsmittel behandelt. Die Temperatur beträgt dabei vorzugsweise 0 bis 30°C, insbesondere etwa 20°C. Die Lösungsmittel-Gewichtsmenge beträgt vorzugsweise das
   1 bis 20-fache, insbesondere 2 bis 10-fache des Ausgangsprodukts. Die Behandlungszeit beträgt vorzugsweise etwa 1 bis 40 Stunden, insbesondere 2 bis 20 Stunden.

Der sich bildende Rückstand wird von der Lösung getrennt und die Lösung von dem Lösungsmittel befreit. Der Rückstand, etwa 30 Gewichtsprozent des Ausgangsproduktes, besteht vorwiegend aus nicht hydroxylierten, langkettigen höheren Fettsäuren die insbesondere etwa eine mittlere Anzahl von 24 Kohlenstoffatomen besitzen.

Der zuvor verbliebene Rückstand, etwa 70 Gewichtsprozent, der die Hydroxyfettsäuren enthält, wird zweckmäßigerweise einmal oder zweimal mit dem unpolaren Lösungsmittel gewaschen. Die Menge beträgt dann etwa 10 Gewichtsprozent der für die Behandlung gewählten Lösungsmittelmenge.

Gegebenenfalls wird der wachsartige Rückstand anschließend vorzugsweise mit einem polaren Lösungsmittel gut vermischt und gerührt. Die Temperatur beträgt dabei vorzugsweise 0 bis 30°C, insbesondere etwa 20°C. Die Lösungsmittel-Gewichtsmenge beträgt vorzugsweise das 0,1 bis 20-fache, insbesondere 0,5 bis 10-fache des Rückstandsgewichtes. Die Behandlungszeit beträgt vorzugsweise 0,5 bis 10 Stunden, insbesondere 1 bis 5 Stunden. Man erhält nach Filtration als Rückstand etwa 60 Gewichtsprozent als Ausgangsmenge einer Mischung, die überwiegend, insbesondere zu mehr als 80 Gewichtsprozent, aus vorwiegend nicht hydroxylierten Fettsäuren mit einer mittleren Anzahl von 14 bis 16 Kohlenstoffatomen besteht. Außerdem können sich in dieser Mischung noch wechselnde Anteile Cholesterin und längerkettige 1,2-Diole befinden.

Die verbliebene Lösung wird vom Lösungsmittel befreit und es verbleibt ein Rückstand von etwa 10 Gewichtsprozent (bezogen auf das Ausgangsgewicht) einer Mischung von Hydroxyfettsäuren.

Zur weiteren Reinigung können die Wollwachssäuren bzw. das Gemisch in an sich bekannter Weise über die Bildung der Salzform gereinigt werden. Dazu werden die Säuren mit einer Base, vorzugsweise Alkalihydroxiden wie NaOH, KOH, in Alkoholen, Wasser, oder Gemischen davon, vorzugsweise aber in Alkoholen wie Methanol oder Ethanol, insbesondere bei pH 12 bis 14, z.B. in alkoholischer KOH-Lösung, in die Salze überführt und gelöst. Feste Verunreinigungen werden abfiltriert.

Anschließend wird mit einer Säure, vorzugsweise mit einer Mineralsäure, vorzugsweise 1/10, bis 1/2-normaler Salzsäure, angesäuert und vorzugsweise so viel Wasser bzw. Säure zugegeben, bis ein pH-Wert von etwa 3 und eine deutliche Phasentrennung vorliegen.

Die sich abscheidende Säure wird abfiltriert oder vorzugsweise mit nicht wassermischbaren Lösungsmitteln wie halogenierten, insbesondere chlorierten Kohlenwasserstoffen, z.B. Methylenchlorid oder aber insbesondere mit Essigsäureethylester extrahiert. Man erhält etwa 10 bis 15 Gewichtsprozent bezogen auf das Ausgangsgewicht eines gereinigten Säuregemisches, insbesondere Hydroxyfettsäuregemisches.

Das erfindungsgemäße Verfahren liefert vorzugsweise und weit überwiegend alpha-Hydroxyfettsäuren. Beta-Hydroxysäuren finden sich abhängig vom Ausgangsprodukt nicht oder nur in sehr geringem Maß. Es treten sowohl geradkettige als auch verzweigte alpha-Hydroxyfettsäuren in wechselnden Anteilen auf. Den Hauptanteil bildet mit vorzugsweise 40 bis 90 Gewichtsprozent alpha-Hydroxyhexadecansäure, bezogen auf den Hydroxyfettsäureanteil.

Daneben finden sich Homologe dieser alpha-Hydroxyfettsäuren mit 12 bis 24 Kohlenstoffatomen, und zwar sowohl normale als auch iso-alpha-Hydroxyfettsäuren in wechselnden Anteilen.

Die aus dem natürlichen Wollwachs stammenden alpha-Hydroxyfettsäuren werden in optisch aktiver Form erhalten. Sie liegen vorzugsweise als D-Enantiomere vor, wie beispielsweise die alpha-Hydroxyhexadecansäure.

Vorzugsweise fällt ein Gemisch von alpha-Hydroxyfettsäuren folgender Zusammensetzung an:

| A. | Gewichtsprozent |
|---|---|
| 1. alpha-Hydroxy-hexadecansäure | 40 - 90 |
| 2. alpha-Hydroxy-16-methylheptadecansäure | 1 - 15 |
| 3. alpha-Hydroxy-tetradecansäure | 1 - 10 |
| 4. alpha-Hydroxy-octadecansäure | 1 - 5 |
| 5. alpha-Hydroxy-dodecansäure | 1 - 2 |
| 6. weitere Homologe von 1. | 1 - 20 |

Besonders bevorzugte Gemische enthalten:

| B. | Gewichtsprozent |
|---|---|
| 1. alpha-Hydroxy-hexadecansäure | 70 - 90 |
| 2. alpha-Hydroxy-16-methylheptadecansäure | 8 - 15 |
| 3. alpha-Hydroxy-tetradecansäure | 2 - 6 |
| 4. alpha-Hydroxy-octadecansäure | 1 - 3 |
| 5. alpha-Hydroxy-dodecansäure | 1 - 2 |
| 6. weitere Homologe von 1. | 1 - 10 |

Das Verfahren liefert vorzugsweise ebenso zwei Klassen nicht hydroxylierter Fettsäuren.

Die Klasse der langkettigen verzweigten und unverzweigten höheren Fettsäuren kann z.B. als unlöslicher Anteil in Schritt 1 (a) des Verfahrens in kristalliner Form isoliert werden.

Es handelt sich vorzugsweise um Fettsäuren mit einer C-Zahl von 18-32, wobei das Verteilungsmaximum bei C-24 liegt.

Die Klasse der kurzkettigen, nicht hydroxylierten höheren Fettsäuren fällt z.B. in Schritt 2 (a) des erfindungsgemäßen Verfahrens in Form löslicher Anteile an, die nach Abdestillation des verwendeten Lösungsmittels in fester oder halbfester Form isoliert werden können. Es handelt sich um verzweigte und unverzweigte Fettsäuren mit einer C-Zahl von 12-18, wobei das Verteilungsmaximum bei C-14 bis C-16 liegt.

Die als Ausgangsprodukt verwendeten bekannten Wollwachssäuregemische sind als natürliche Produkte unterschiedlich zusammengesetzt. Zweckmäßigerweise werden sie in gereinigter Form beispielsweise in destillierter Form eingesetzt, nachdem sie in üblicher Weise durch Verseifung des Wollwachses erhalten wurden. Besonders bevorzugt können Wollwachssäuregemische aus der Spaltung von Wollfett australischer und neuseeländischer Herkunft verwendet werden.

Die verwendeten Lösungsmittel werden vorzugsweise rein oder technisch rein eingesetzt und sind weitgehend wasserfrei. Es können einzelne Lösungsmittel oder auch Gemische, vorzugsweise von 2 bis 5 Lösungsmitteln, insbesondere 2 oder 3 Lösungsmitteln verwendet werden.

Die Ausgangsprodukte und überraschenderweise auch die in den Zwischenstufen des Verfahrens erhaltenen Säuregemische liegen zumeist wachsartig bis kristallin vor, und sie lassen sich unter üblichen Verfahrensbedingungen und Einsatz üblicher Mechanik wirkungsvoll bearbeiten, insbesondere bei Raumtemperatur.

Alle Prozentangaben oder Teile beziehen sich immer, soweit nicht anders angegeben, auf das Gewicht, insbesondere auf das Gesamtgewicht der Zubereitungen.

Es stellte sich überraschenderweise heraus, daß alpha-Hydroxyfettsäuren eine hohe antimikrobielle Wirksamkeit gegenüber Hautbakterien besitzen, wie der folgende Versuchsbericht zeigt.

Synthetisch hergestellte Hydroxyfettsäuren (siehe A. H. Eggerth) liegen üblicherweise als Racemat vor. Nach der Trennung des Racemats in die beiden Enantiomeren stellte sich überraschenderweise heraus, daß beide Enantiomeren jeweils noch höhere Aktivität besaßen als das Racemat. Dieses Ergebnis wurde zusätzlich dadurch bestätigt, daß die erneute Vermischung der Enantiomeren wieder zu der Aktivität führte, wie sie bei dem Racemat beobachtet wurde.

Es wurden verschiedene alpha-Hydroxyfettsäuren getestet. Alle zeigten eine sehr hohe antimikrobielle Wirksamkeit, die weit über dem als Vergleichssubstanz mitgetesteten GML lagen.

### I: Testorganismus Staphylococcus epidermidis Testkonzentration: 2 mmol

| | KBE/ml | | |
|---|---|---|---|
| | 1h | 3h | 19h |
| alpha-OH-Hexadecansäure (D) | < 10² | < 10² | < 10² |
| alpha-OH-Hexadecansäure (L) | < 10² | < 10² | < 10² |
| Kontrolle | 5,9x10⁶ | 1,1x10⁷ | 9,7x10⁷ |
| (GML) Glycerinmonolaurat (10 mmol) | 2,9x10⁴ | 3,1x10⁴ | 6,5x10³ |
| (KBE = Kolonien bildende Einheiten) | | | |

Bezüglich der Deowirksamkeit stellen die Coryneformen Bakterien die wichtigste Gruppe von Bakterien dar, da sie für die Entstehung des Achselgeruchs verantwortlich sind.

### II. Testorganismus: Corynebakterium xerosis; Ausgangstiter 1x10⁵ KBE/ml Inkubationszeit: 19 h Konzentration: 10 mM

| | Reduktionsfaktor (im Vergleich zur Kontrolle) |
|---|---|
| alpha-Hydroxy-stearinsäure (R) | 10⁵ |
| alpha-Hydroxy-palmitinsäure (R) | 10⁴ |
| alpha-Hydroxy-myristinsäure (R) | 10³ |
| alpha-Hydroxy-laurinsäure (R) | 10³ |
| (R = Racemat) | |
| GML (20mM) | 10¹ |

Die Versuche wurden wie folgt durchgeführt:

Eine frische Übernachtkultur des Testorganismus wurde 1:10⁴ in Nährmedium (5 g/l Tryptone ^{R}, 3 g/l Hefeextrakt, pH 7) verdünnt und 3 h inkubiert (30° C, 250 rpm). Anschließend wurden die Testsubstanzen in der angegebenen Endkonzentration zugegeben und die Inkubation fortgesetzt. Nach bestimmten Zeitintervallen wurden Proben entnommen und auf den Lebendtiter (siehe Tabellen, KBE/ml) überprüft. Aus der Differenz zum Titer der Kontrolle, die nur Bakterien aber keine Fettsäure enthielt, ergab sich die antimikrobielle Wirksamkeit.

Es wurde gefunden, und auch darin liegt die Lösung der Aufgabe gemäß der Erfindung, daß man unter Verwendung der üblichen kosmetischen Trägerstoffe zu desodorierenden kosmetischen Mitteln mit sehr guten desodorierenden Eigenschaften, gelangt, wenn man derartigen Mitteln geringe Mengen von den erfindungsgemäßen desodorierend wirkenden alpha-Hydroxyfettsäuren zusetzt.

Gegenstand der Erfindung ist somit auch die Verwendung eines Wirkstoffes oder mehrerer Wirkstoffe in einem desodorierenden kosmetischen Mittel auf Basis üblicher kosmetischer Trägerstoffe, das dadurch gekennzeichnet ist, daß es als Wirkstoff die genannten desodorierenden alpha-Hydroxyfettsäuren enthält.

Vorzugsweise soll der Gehalt des erfindungsgemäßen desodorierenden kosmetischen Mittels an desodorierenden alpha-Hydroxyfettsäuren oder deren Salzen 0,01 bis 10 Gewichts-%, bezogen auf die Gesamt-Zusammensetzung des Mittels oder der Zubereitung, betragen, insbesondere aber 0,1 bis 1 Gewichtsprozent, besonders bevorzugt aber 0,25 bis 0,75 Gewichtsprozent.

Das desodorierende kosmetische Mittel gemäß der Erfindung kann in Form eines aus Aerosolbehältern, Quetschflaschen oder durch eine Pumpvorrichtung versprühbaren Präparates vorliegen oder in Form einer mittels Roll-on-Vorrichtungen auftragbaren flüssigen Zusammensetzung, jedoch auch in Form einer aus normalen Flaschen und Behältern auftragbaren W/O- oder O/W-Emulsion, z.B. einer Creme oder Lotion. Weitere kosmetische Desodorantien können in Form von Deo-Sprays, Deo-Roll-ons, Deo-Pumpsprays, desodorierenden Tinkturen, desodorierenden Intimreinigungsmittel, desodorierenden Shampoos, Deo-Seife, desodorierenden Dusch- oder Badezubereitungen, desodorierenden Pudern oder desodorierenden Pudersprays vorliegen. Es können aber auch übliche Deo-Stift-Grundmassen als Träger für feste Zubereitungen und Stifte dienen.

Gegenstand der Erfindung ist auch die Verwendung von Zusammensetzungen mit einem wirksamen Gehalt an alpha-Hydroxyfettsäuren als kosmetische Desodorantien.

Als übliche kosmetische Trägerstoffe zur Herstellung der desodorierenden Mittel gemäß der Erfindung können neben Wasser, Äthanol und Isopropanol, Glycerin, und Propylenglykol hautpflegende Fett- oder fettähnliche Stoffe, wie Partialglyceride von Fettsäuregemischen, Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, wie Methylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Treibmittel für aus Aerosolbehältern in Form eines Sprühstrahls bei Betätigung des Ventils versprühbare desodorierende kosmetische Mittel gemäß der Erfindung sind die üblichen bekannten leichtflüchtigen, verflüssigten Treibmittel, wie Kohlenwasserstoffe (Propan, Butan, Isobutan) und chlorfluorierte Kohlenwasserstoffe (Dichlordifluormethan, Trichlormonofluormethan, Dichlortetrafluoräthan) geeignet, die allein oder in Mischung miteinander eingesetzt werden können.

Als Emulgatoren zur Herstellung der erfindungsgemäßen desodorierenden kosmetischen Mittel, die vorzugsweise als flüssige Zubereitungen mittels einer Roll-on-Vorrichtung auf die gewünschten Hautbereiche aufgetragen werden sollen, und die in den Mitteln in geringer Menge (z.B.) 2 bis 5 Gewichts.-%, bezogen auf die Gesamt-Zusammensetzung, verwendet werden können, haben sich nichtionogene Typen, wie Polyoxyäthylen-Fettalkoholäther, z.B. Cetostearylalkoholpolyäthylenglykoläther mit 12 bzw. 20 angelagerten Äthylenoxid-Einheiten pro Molekül Cetostearylalkohol, sowie Sorbitanester und Sorbitanester-Äthylenoxid-Verbindungen (z.B. Sorbitanmonostearat und Polyoxyäthylensorbitanmonostearat) als auch langkettige höhermolekulare wachsartige Polyglykoläther als geeignet erwiesen.

Zusätzlich zu den genannten Bestandteilen können den desodorierenden kosmetischen Mitteln gemäß der Erfindung, deren pH-Wert vorzugsweise z.B. durch übliche Puffergemische auf 5 bis 7,5, insbesondere 6 bis 7,5, eingestellt wird, kleine Mengen Parfüm, Farbstoffe, Antioxidantien (z.B. "Ionol"=2,6-Di-Tert.-butyl-4-methylphenol in Mengen von 0,01 bis 0,03%, bezogen auf die Gesamt-Zusammensetzung), Suspendiermittel, Puffergemische oder andere übliche kosmetische Grundstoffe, wie Triäthanolamin oder Harnstoff, beigemischt werden. Für Deo-Stifte und Deo-Seife wird ein pH-Bereich von 8,5 bis 9,8 bevorzugt. Insgesamt wird ein pH-Bereich von 5 bis 10 bevorzugt.

Zur Parfümierung sind insbesondere solche Substanzen und Parfümöle geeignet, die stabil sind, die Haut nicht reizen und bereits als solche antibakterielle (bakteriostatische) Eigenschaften besitzen.

Die Erfindung ist aber nicht auf die genannten Zubereitungen und Mittel, Hilfsstoffe und Trägersubstanzen beschränkt.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen Mittel, die auch Gegenstand der Erfindung ist, erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Die erfindungsgemäßen desodorierenden alpha-Hydroxy-Fettsäuren oder deren Salze können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

Vorteilhaft ist es auch, sie in sogenannte Pudersprays einzuarbeiten. Sollen die erfindungsgemäßen Kombinationen in Pudersprays eingearbeitet werden, so können die Suspensionsgrundlagen dafür vorteilhaft gewählt werden aus der Gruppe Aerosil, Kieselgur, Kaolin, Talkum, modifizierte Stärke, Titandioxid, Seidenpulver, Nylonpulver, Polyethylenpulver und verwandten Stoffen.

Alle Prozentangaben oder Teile beziehen sich, soweit nicht anders angegeben, auf das Gewicht, insbesondere auf das Gesamtgewicht der Zubereitungen.

Zum Nachweis des überraschenden desodorierenden Effektes der erfindungsgemäßen alpha-Hydroxysäuren wurde die folgende Untersuchung durchgeführt.

Es wurde eine erfindungsgemäße Zusammensetzung gegen Placebo, also bis auf den Gehalt an Wirksubstanz identischen Zusammensetzungen getestet. Ein Kollektiv von 30 Probanden hatte die Auflage, jeweils eine Achselhöhle mit erfindungsgemäßer Zusammensetzung und die andere mit Placebo zu behandeln. Die Probanden trugen daraufhin drei Stunden lang ein Hemd mit Slipeinlagen unter den Achseln. Nach Ablauf dieser Zeit wurden die Slipeinlagen in separate Flaschen überführt. Der Geruch der Einlagen wurde von drei Prüfpersonen beurteilt. Der Versuch wurde als Doppelblindversuch durchgeführt, so daß weder die Probanden noch die Prüfpersonen wußten, welche Achsel mit welcher Zusammensetzung behandelt worden war. Die Bestimmung der Geruchsentwicklung wurde im Testzeitraum von 4 Wochen mehrfach wiederholt.

Es stellte sich heraus, daß die wirkstoffhaltige Zubereitung in jeweils 62 von 111 Fällen subjektiv besser wirkte als das entsprechende Placebo. In 34 von 111 Fällen gaben die Prüfpersonen an, behandelte und unbehandelte Proben würden sich nicht voneinander unterscheiden. In 15 Fällen wurde unter der mit Placebo behandelten Achsel die geringere Geruchsentwicklung festgestellt.

Untersucht wurde die folgende Zusammensetzung:

| | |
|---|---|
| alpha-Hydroxypalmitinsäure | 5 g |
| Ethanol pharm. (96%) | 350 g |
| Wasser | auf 1 000 g |

Die Erfindung wird anschließend anhand von Beispielen näher erläutert. Die gemäß den folgenden Beispielen hergestellten Präparate zeigten in jedem Falle neben einer ausgezeichneten Haut- und Schleimhautverträglichkeit bei bestimmungsmäßer Anwendung eine sehr gute und lang anhaltende desodorierende Wirkung.

### Konservierungshelfer

Bisher waren keine natürlich vorkommenden Fettsäuren bekannt, die sowohl gegen grampositive als auch gegen gramnegative Bakterien gleichermaßen und im sauren sowie alkalischen Bereich gut wirksam sind.

Lediglich die Amine bestimmter Fettsäuren zeigten eine antimikrobielle Wirksamkeit sowohl gegen grampositive als auch gegen gramnegative Bakterien (J.J. Kabara (1972), "Fatty Acids and Derivatives as Antimicrobial Agents", Antimicrob. Agents Chemother. 2, 23 - 28).

Alpha-Hydroxyfettsäureseifen besitzen bekanntlich eine germizide Wirkung gegenüber einigen grampositiven, aber auch gegenüber dem gramnegativen Organismus B. pyocyaneus (A. H. Eggerth, siehe oben), der heute Pseudomonas aeruginosa genannt wird. Aus der dort angegebenen Tabelle I (Seite 303) ist zu entnehmen, daß mit steigendem pH-Wert von pH 7 auf pH 7,5 eine höhere Wirksamkeit gegenüber diesem Organismus verbunden ist, während alle getesteten alpha-Hydroxy-Fettsäuren schon bei pH 6,5, d.h. im schwach sauren Bereich, unwirksam waren.

Aufgabe der Erfindung ist es, als Konservierungshelfer Stoffe bereitzustellen und kosmetische Mittel und Zubereitungen mit Konservierungshelfern zu schaffen, die die Nachteile des Standes der Technik nicht aufweisen und insbesondere einen im Hinblick auf die Wirksamkeit, Hautschonung und Verträglichkeit verbesserten Wirkstoff zur Verfügung zu stellen sowie kosmetische Zubereitungen, die diesen Konservierungshelfer enthalten.

Diese Aufgabe wird gelöst durch die Verwendung von alpha-Hydroxyfettsäuren mit 10 bis 24 Kohlenstoffatomen oder deren Salzen als Konservierungshelfer in kosmetischen Zubereitungen, wobei die alpha-Hydroxyfettsäuren sowohl einzeln als auch im Gemisch vorliegen können.

Bevorzugt werden kosmetische Zubereitungen und Mittel und bevorzugt werden kosmetisch verträgliche Salze, insbesondere Alkali- oder Ammoniumsalze, beispielsweise Natriumsalze oder Kaliumsalze.

Gemische können von Zwei, mehreren aber auch einer größeren Anzahl von Verbindungen gebildet werden und diese Verbindungen in unterschiedlichen Gewichtsmengen enthalten. So können aus natürlichen Quellen stammende Fraktionen eine größere Zahl aufgrund von Homologen oder Strukturisomeren aufweisen, synthetisierte Säuren können dagegen aus jeweils einer Verbindung bestehen. Erfindungsgemäß soll mindestens eine der genannten alpha-Hydroxysäuren verwendet werden oder in der Zubereitung enthalten sein.

Die erfindungsgemäßen alpha-Hydroxyfettsäuren können geradkettig oder verzweigt sein. Bevorzugt werden die gesättigten aliphatischen alpha-Hydroxyfettsäuren.

Es wurde überraschenderweise gefunden, daß die erfindungsgemäßen alpha-Hydroxyfettsäuren und ihre Salze hervorragende Konservierungshelfer darstellen.

Durch die Hydroxygruppe erhalten die Fettsäuren mindestens ein asymmetrisches Kohlenstoffatom, und sie können als Racemat, Diastereoisomere oder in der optisch aktiven D- oder L-Form vorliegen. Bevorzugt werden entweder die Verwendung der D-Form oder die der L-Form sowie Zubereitungen, die entweder die D-Form oder die L-Form enthalten.

Bevorzugte erfindungsgemäße als Konservierungshelfer wirkende alpha-Hydroxyfettsäuren oder deren Salze enthalten 12 bis 20 Kohlenstoffatome, insbesondere aber 12 bis 18 Kohlenstoffatome. Vorteilhaft sind auch Gemische der in solchen Bereichen liegenden Fettsäuren.

Besonders bevorzugt sind die folgenden Fettsäuren und ihre Salze:
alpha-Hydroxy-hexadecansäure
   (alpha-Hydroxy-palmitinsäure)
alpha-Hydroxy-stearinsäure
alpha-Hydroxy-16-methylheptadecansäure
alpha-Hydroxy-laurinsäure
   oder
alpha-Hydroxy-myristinsäure.

Ganz besonders bevorzugt werden die optischen Isomere der vorstehend genannten Hydroxyfettsäuren und Gemische, d.h. jeweils der D- und L-Form, insbesondere aber der D-Form.

Erfindungsgemäß können vorteilhaft aus Wollwachs gewonnenene alpha-Hydroxyfettsäuren eingesetzt werden, z.B. auch gemäß vorstehendem Isolierungsverfahren erhältliche alpha-Hydroxy-Fettsäuregemische, die aus Wollwachssäuregemischen gewonnen werden. Sie fallen vorzugsweise als D-Enantiomeren-Gemisch an. Auch die nach bekannten Verfahren daraus erhältlichen Einzelverbindungen sind bevorzugt.

Die erfindungsgemäßen alpha-Hydroxyfettsäuren sind bekannt oder nach bekannten Verfahren erhältlich. In der Synthese anfallende Racemate lassen sich in üblichen Verfahren in die Isomeren aufspalten.

Es wurde überraschenderweise gefunden, daß die erfindungsgemäßen alpha-Hydroxyfettsäuren als Konservierungshelfer wirken und daß, entgegen der Lehre des Standes der Technik, sogar im sauren pH-Bereich, insbesondere im Bereich von pH 5 - 6,5 gegenüber gramnegativen Organismen eine ebenso hohe antimikrobielle Wirksamkeit vorhanden war, wie bei pH 7,5. Diese Eigenschaft ist aber für Konservierungshelfer sehr vorteilhaft.

Die beiden optisch aktiven Enantiomeren sind noch wirksamer als das Racemat. Dieses Ergebnis wurde zusätzlich dadurch bestätigt, daß die erneute Vermischung der Enantiomeren wieder zu der Aktivität führte, wie sie bei dem Racemat beobachtet wurde.

Im folgenden Versuchsbericht wird die hohe Wirksamkeit gegenüber gramnegativen Organismen bei verschiedenen Konzentrationen und die Überlegenheit im Vergleich mit Glycerin-mono-laurat (GML) nachgewiesen.

### I. Wirksamkeit

### a) Testorganismus Pseudomonas aeruginosa Testkonzentration: 6 mmol

| | KBE/ml | | |
|---|---|---|---|
| | 1h | 3h | 19h |
| alpha-OH-Hexadecansäure (R) | < 10² | < 10² | < 10² |
| alpha-OH-Tetradecansäure (R) | < 10² | < 10² | < 10² |
| alpha-OH-Dodecansäure (R) | < 10² | < 10² | < 10² |
| alpha-OH-Decansäure (R) | 3,2x10⁵ | 1,9x10⁴ | 2,9x10⁷ |
| Kontrolle | 2,3x10⁶ | 2,1x10⁷ | 3,8x10⁹ |
| Glycerinmonolaurat (20 mmol) | 2,9x10⁶ | 9,5x10⁶ | 8,7x10⁹ |
| Laurinsäure | 3,6x10⁶ | 6,6x10⁶ | 1,0x10⁹ |
| (R = Racemat) (KBE = Kolonien bildende Einheiten) | | | |

### Beispiel b) Testorganismus Pseudomonas aeruginosa Testkonzentration: 3 mmol

| | KBE/ml | | |
|---|---|---|---|
| | 1h | 3h | 19h |
| alpha-OH-Hexadecansäure (D) | 1,1x10² | 1,1x10² | 1,3x10⁴ |
| alpha-OH-Hexadecansäure (L) | 6,5x10² | 2,2x10² | 1,7x10⁵ |
| Kontrolle | 2,3x10⁶ | 1,2x10⁷ | 4,9x10⁹ |
| (KBE = Kolonien bildende Einheiten) | | | |

Eine frische Übernachtkultur des Testorganismus wurde 1:10⁴ in Nährmedium (5 g/l Tryptone^{R}, 3 g/l Hefeextrakt, pH 7) verdünnt und 3h inkubiert (30° C, 250 rpm). Anschließend wurden die Testsubstanzen in der angegebenen Endkonzentration zugegeben und die Inkubation fortgesetzt. Nach bestimmten Zeitintervallen wurden Proben entnommen und auf den Lebendtiter überprüft. Aus der Differenz zum Titer der Kontrolle, die nur Bakterien aber keine Fettsäure enthielt, ergab sich die antimikrobielle Wirksamkeit.

### II. Wirkungsspektrum

Die Wirksamkeit der alpha-Hydroxyfettsäuren erstreckte sich nicht nur auf Pseudomonas aeruginosa sondern auch auf andere gramnegative Mikroorganismen. Dies wurde gezeigt durch die Bestimmung der minimalen Hemmkonzentration für die entsprechenden Bakterien.

Die hervorragende antibakterielle Wirkungsstärke der erfindungsgemäßen alpha-Hydroxyfettsäuren ist auch aus der niedrigen minimalen Hemmkonzentration ersichtlich, die für einige Organismen angegeben wird. Es wurde alpha-Hydroxy-palmitinsäure untersucht.

Bestimmung der minimalen Hemmkonzentration Eine frische Übernachtkultur des Testorganismus wurde 1:10⁴ in Nährmedium (5 g/ltr Tryptone, 3 g/ltr Hefeextrakt, pH 7,0) verdünnt und 3h inkubiert (30° C, 250 rpm). Anschließend wurde alpha-Hydroxy-palmitinsäure (D) in einer Endkonzentration von 2,0%; 1,5%; 1,0%; 0,75%; 0,5%; 0,25%; 0,1% und 0,06% zugegeben. Nach 72 h Inkubationszeit wurden der Bakterientiter durch Verdünnung und Ausplattierung überprüft. Bei den Ansätzen, bei denen keine Bakterien nachweisbar waren, wurde 1 ml des Ansatzes mit 9 ml Nährmedium versetzt und über Nacht inkubiert (30° C, 250 rpm). Als minimale Hemmkonzentration wurde die geringste Fettsäurekonzentration angesehen, bei der in dem betreffenden Ansatz keine Bakterien mehr nachweisbar waren. Es werden folgende Ergebnisse erhalten:

Minimale Hemmkonzentration grampositiver Bakterien:

| | |
|---|---|
| Staphylococcus epidermidis | 0,10% |
| Corynebacterium xerosis | 0,10% |
| Micrococcus luteus | 0,25% |

Minimale Hemmkonzentration gramnegativer Bakterien:

| | |
|---|---|
| Pseudomonas aeruginosa | 0,25% |
| Pseudomonas putida | 0,10% |
| Escherichia coli | 0,50% |
| Klebsiella pneumoniae | 0,50% |
| Salmonella livingstone | 0,50% |
| Acinetobacter calcoaceticus | 0,75% |

Mit dem alpha-Hydroxyfettsäuregemisch des Beispiels 1 wurden gleiche Ergebnisse erhalten.

### III. pH-Abhängigkeit

Die hohe antimikrobielle Wirksamkeit unter sauren sowie alkalischen Bedingungen gegen gramnegative Organismen ist von großer Bedeutung für die Anwendung als Konservierungshelfer in Kosmetika, die mit der Haut in Berührung kommen, da hierfür schwach saure pH-Bereiche eingestellt werden oder natürlicherweise vorliegen. Erst durch diese überraschend festgestellten Eigenschaften werden solche Anwendungen möglich.

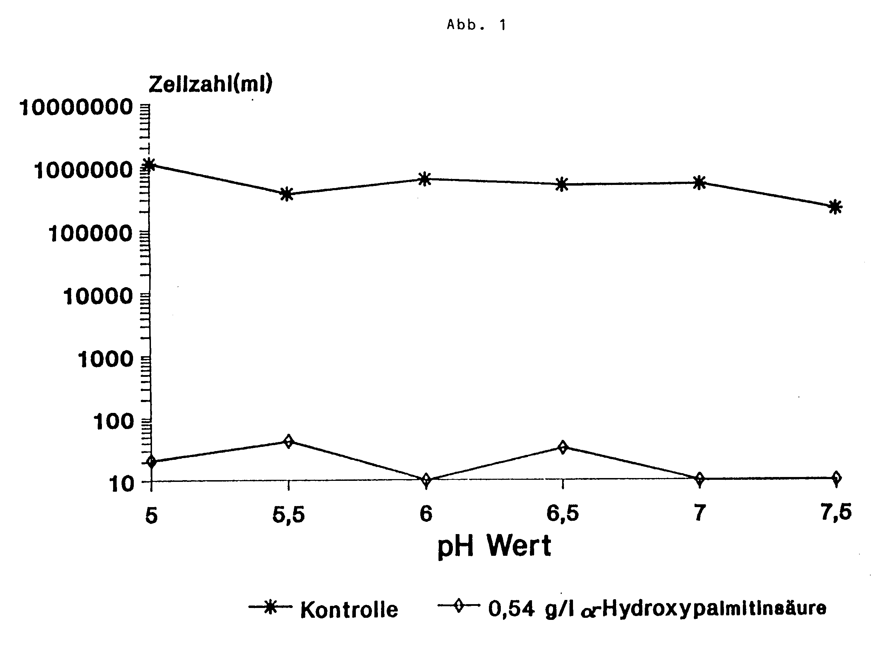

Aus der Abbildung 1 ist die pH-Abhängigkeit der Wirkungstärke gegenüber gramnegativen Organismen, z.B. für Pseudomonas aeruginosa gezeigt. Es besteht eine hervorragende Wirkung der erfindungsgemäßen alpha-Hydroxyfettsäuren im sauren Bereich.

Zur Ermittlung der Versuchsergebnisse wurde eine frische Übernachtkultur von Pseudomonas aeruginosa 1:10⁴ in Nährmedium (86 g/l Tryptone^{R}, 3 g/l Hefeextrakt, pH 7) verdünnt und inkubiert (30°C, 250 rpm). Nach dem Erreichen eines Bakterientiters von 10⁶ Bakterien/ ml wurde 1 ml Kultur entnommen und die Bakterien wurden abzentrifugiert (5000 rpm, 5 min), der Überstand wurde verworfen und das Bakterienpellet in 1 ml 50mM Tris-Puffer bei den genannten pH-Werten mit 0,54 mg alpha-Hydroxy-palmitinsäure resuspendiert. Nach einer Inkubation von 30 min (RT) wurde die Bakterienkultur abzentrifugiert, der Überstand verworfen, das Pellet in 1 ml Inhibierungspuffer (3% Tween 80, 0,3% Lecithin, 0,1% Histidin) resuspendiert und auf Nährböden zur Bestimmung des Lebendtiters ausplattiert.

Als Konservierungshelfer können die alpha-Hydroxyfettsäuren gemäß der Erfindung in kosmetischen Zubereitungen wie kosmetischen Rohstoffen, kosmetischen Vorprodukten, und insbesondere in kosmetischen Mitteln verwendet werden.

Die kosmetischen Zubereitungen gemäß der Erfindung sind daher auch kosmetische Rohstoffe, kosmetische Vorprodukte und kosmetische Mittel mit den erfindungsgemäßen als Konservierungshelfer wirkenden alpha-Hydroxyfettsäuren. Geeignet sind alle üblichen kosmetischen Zubereitungen bzw. Mittel oder Kosmetika.

Die erfindungsgemäßen kosmetischen Mittel können wie üblich zusammengesetzt sein und zur Behandlung der Haut und/oder der Haare und als Schminkprodukt in der dekorativen Kosmetik dienen.

Vorzugsweise soll der Gehalt der kosmetischen Zubereitungen und des erfindungsgemäßen kosmetischen Mittels an konservierenden alpha-Hydroxyfettsäuren oder deren Salzen 0,01 bis 10 Gewichts-%, bezogen auf die Gesamt-Zusammensetzung der Zubereitung und des Mittels betragen, insbesondere aber 0,1 bis 2,5 Gew.-%, besonders bevorzugt aber 0,25 bis 1,5 -Gew.-%.

Als zweckmäßig hat es sich erwiesen, den pH-Wert der Zubereitungen und der kosmetischen Formulierung im Bereich von 4,5 bis 9 einzustellen, insbesondere aber im Bereich von pH 5 bis pH 7.

Gegenstand der Erfindung sind auch die Verwendung eines Wirkstoffes oder mehrerer Wirkstoffe in kosmetischen Zubereitungen, insbesondere kosmetischen Mitteln auf Basis üblicher kosmetischer Trägerstoffe, wobei der Wirkstoff eine erfindungsgemäße als Konservierungshelfer wirkende alpha-Hydroxyfettsäure ist.

Geeignet sind beispielsweise die bekannten kosmetischen Zubereitungen wie O/W-Emulsionen, W/O-Emulsionen, Cremes, Lotionen, Milchen, Salben, Fettsalben, Gele, Tinkturen, Solubilisate, Lösungen, Suspensionen, ölige Zubereitungen oder feste, beispielsweise stiftförmige Zubereitungen; Puder oder Sprays in allen Formen.

Die Kosmetika können in bekannter Weise Trägerstoffe, Hilfsstoffe und Zusatzstoffe enthalten. Es sind z.B. wasserbindende Stoffe, Verdicker, Füllstoffe, Parfüm, Farbstoffe, Tenside, Lichtschutzmittel, Öle, Fette, Wachse, Sonnenschutzmittel, UVA-Blocker, UVB-Blocker, Vitamine, Proteine, Insektenrepellentien, Stabilisatoren, Antioxidantien, Konservierungsmittel, Alkohole, keratolytisch oder proteolytisch wirksame Substanzen, Wasser und/oder Salze, beispielsweise Puffersalze oder Puffergemische zur pH-Einstellung.

Kosmetische Rohstoffe und Vorprodukte sind beispielsweise Einzelverbindungen, Mischungen oder Konzentrate von kosmetischen Trägerstoffen, Hilfsstoffen oder Wirkstoffen und unterscheiden sich daher nicht wesentlich von kosmetischen Mitteln, so daß auf diese bezogene Angaben sich gleichermaßen auf solche Rohstoffe oder Vorprodukte beziehen.

Bevorzugte erfindungsgemäße Kosmetika sind wasserhaltige Mittel wie WAS-Zubereitungen, z.B. Lösungen, Suspensionen, Dispersionen oder Gele, insbesondere auf Wasserbasis oder Wasser-Alkohol-Basis.

Besonders bevorzugt sind Badepräparate, Schaumbäder, Duschgele und Shampoos.

Der erfindungsgemäße Konservierungshelfer zeichnet sich durch eine gute Hautverträglichkeit und eine gute Wirkung aus.

Die Erfindung wird in den Beispielen erläutert. Auch die hergestellten Präparate besitzen eine gute Wirkung und Verträglichkeit.

Zur Parfümierung sind insbesondere solche Substanzen und Parfümöle geeignet, die stabil sind und die Haut nicht reizen.

Die Erfindung ist aber nicht auf die genannten Zubereitungen und Mittel, Hilfsstoffe und Trägersubstanzen beschränkt.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden die Fettphase und die Wasserphase z.B. separat gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Die erfindungsgemäßen alpha-Hydroxy-Fettsäuren oder deren Salze können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

### Ionen-Bindungsvermögen und Ionen-Komplexierungsvermögen

Alpha-Hydroxyfettsäuren zeichnen sich überraschenderweise weiter durch ein hohes Komplexierungs- und Bindungsvermögen für Metallionen aus.

Metallionen wie Erdalkali und insbesondere Schwermetallionen können in Kosmetika zu unerwünschten Reaktionen wie Verfärbungen führen. Sie reagieren mit anionischen Bestandteilen von kosmetischen Formulierungen zu unlöslichen störenden Komplexen. Die Oxidation von Farb- und Riechstoffen wird durch Spuren von Schwermetallen katalysiert. Die Wirksamkeit von antimikrobiell wirksamen Substanzen wird durch die Anwesenheit dieser Kationen beeinträchtigt. Komplexbildende Substanzen wie z.B. EDTA finden daher in Kosmetik weite Anwendung.

Komplexbildner wie NTA, insbesondere EDTA, besitzen aber den Nachteil, daß sie biologisch schwer abbaubar sind und daher in zunehmenden Maße eine Umweltbelastung darstellen. Es ist bekannt, daß einige kurzkettige alpha-Hydroxycarbonsäuren und auch einige Fettsäuren ein gewisses Ca²⁺-Bindungspotential besitzen. Entsprechende Komplexbildungskonstanten sind für Laktat, Mandelat, Tartrat, Malat und Citrat verfügbar. Diese Säuren bilden aber nicht die für die Verwendung in Kosmetika erforderlichen stabilen Komplexe.

Bekannt ist auch, daß Kupfersalzlösungen mit alpha-Hydroxyfettsäuren Ausfällungen ergeben.

Aufgabe der Erfindung war es, für Kosmetika einen Metallionen bindenden oder komplexierenden Stoff zur Verfügung zu stellen, der die Nachteile des Standes der Technik nicht hat und insbesondere geeignet ist, EDTA in Kosmetika zu ersetzen.

Diese Aufgabe wird gelöst durch die Verwendung von alpha-Hydroxyfettsäuren mit 12 bis 24 Kohlenstoffatomen oder deren Salzen zum Binden oder Komplexieren von Ionen in kosmetischen Zubereitungen , wobei die alpha-Hydroxyfettsäuren sowohl einzeln als auch im Gemisch vorliegen können.

Für Kosmetika werden kosmetisch verträgliche Salze, insbesondere Alkali oder Ammoniumsalze, beispielsweise Natriumsalze oder Kaliumsalze, bevorzugt.

Gemische können von zwei, mehreren aber auch einer größeren Anzahl von Verbindungen gebildet werden und diese Verbindungen in unterschiedlichen Gewichtsmengen enthalten. So können aus natürlichen Quellen stammende Fraktionen eine größere Zahl aufgrund von Homologen oder Strukturisomeren aufweisen, synthetisierte Säuren können dagegen aus jeweils einer Verbindung bestehen. Erfindungsgemäß soll mindestens eine der genannten alpha-Hydroxysäuren verwendet werden oder in der Zubereitung enthalten sein.

Die erfindungsgemäßen alpha-Hydroxyfettsäuren können geradkettig oder verzweigt sein. Bevorzugt werden die gesättigten, aliphatischen alpha-Hydroxyfettsäuren. Es wurde überraschenderweise gefunden, daß die erfindungsgemäßen alpha-Hydroxyfettsäuren und ihre Salze eine ausgeprägte ionenbindende oder ionenkomplexierende Wirkung besitzen.

Bevorzugte erfindungsgemäße ionenbindende und komplexierende alpha-Hydroxyfettsäuren oder deren Salze enthalten 14 bis 20 Kohlenstoffatome, insbesondere aber 14 bis 18 Kohlenstoffatome. Vorteilhaft sind auch Gemische der in solchen Bereichen liegenden Fettsäuren.

Besonders bevorzugt sind die folgenden Fettsäuren und ihre Salze:
alpha-Hydroxy-hexadecansäure
   (alpha-Hydroxy-palmitinsäure)
alpha-Hydroxy-myristinsäure
alpha-Hydroxy-16-methylheptadecansäure
   oder
alpha-Hydroxy-stearinsäure.

Erfindungsgemäß können vorteilhaft aus Wollwachs gewonnene alpha-Hydroxyfettsäuren eingesetzt werden, z.B. auch gemäß vorstehendem Isolierungsverfahren erhältliche alpha-Hydroxy-Fettsäuregemische, die aus Wollwachssäuregemischen gewonnen werden. Sie fallen vorzugsweise als D-Enantiomeren-Gemisch an. Auch die nach bekannten Verfahren daraus erhältlichen Einzelverbindungen sind bevorzugt.

Die erfindungsgemäßen alpha-Hydroxyfettsäuren sind bekannt oder nach bekannten Verfahren erhältlich. In der Synthese anfallende Racemate lassen sich in üblichen Verfahren in die Isomeren aufspalten.

Vorzugsweise werden erfindungsgemäß Metallionen, z.B. Erdalkaliionen und Schwermetallionen gebunden oder komplexiert.

Besonders bevorzugte Ionen sind Ca²⁺, Mg²⁺, Fe²⁺, Fe³⁺, Cu¹⁺, Cu²⁺, Mn²⁺, Hg¹⁺, Hg²⁺.

Die erfindungsgemäßen alpha-Hydroxyfettsäuren haben gegenüber Schwermetallionen, z.B. Fe²⁺ oder Erdalkali-Ionen wie Ca²⁺ eine hohe Bindungsfähigkeit, wie die folgende Untersuchung zeigt:

### a) Konzentration an freiem Ca²⁺ nach Zugabe von 1 mmol Testsubstanz

| | Konz. Ca²⁺ (nmol) |
|---|---|
| Kontrolle (ohne Fettsäure) | 200 |
| alpha-Hydroxy-myristinsäure | 32 |
| Myristinsäure | 233 |
| alpha-Hydroxypalmitinsäure | 38 |
| Palmitinsäure | 249 |
| alpha-Hydroxyfettsäuregemisch des Beispiels 1 | 44 |
| EDTA | 25 |
| Citronensäure | 139 |
| Mandelsäure | 200 |

Der Testpuffer (100 mM KCl, 10 mM HEPES, 1 mM EGTA 0,5 mM CaCl₂, pH 7,1) wurden mit je 1 mM der Testsubstanz versetzt. Entstehende Niederschläge wurden abfiltriert. Das Filtrat wurde mit KOH auf pH 7,1 eingestellt und mit 1 µM FURA 2 versetzt. Die Konzentration an Ca²⁺ wurde fluorimetrisch bestimmt (Anregung 340 nm, 359 nm, 380 nm, Emission 510 nm).

### b) Konzentration an freiem Fe²⁺ nach Zugabe von 1 mmol alpha-Hydroxypalmitinsäure

| | Konz. Fe²⁺ (µmol) |
|---|---|
| Citronensäure | 110 |
| Mandelsäure | 100 |
| alpha-Hydroxypalmitinsäure | 20 |

1 mmol Testsubstanz wurde mit 1 mmol Fe²⁺ versetzt. Entstehende Niederschläge wurden abfiltriert. Die Filtrate wurden mit 2,2'-Bipyridin versetzt und bei 565 nm photometrisch vermessen.

Gegenstand der Erfindung ist die Verwendung der erfindungsgemäßen ionenbindenden und ionenkomplexierenden alpha-Hydroxyfettsäuren in kosmetischen Zubereitungen Geeignet sind alle üblichen kosmetischen Zubereitungen bzw. Mittel oder Kosmetika.

Die erfindungsgemäßen kosmetischen Mittel können wie üblich zusammengesetzt sein und zur Behandlung der Haut und/oder der Haare und als Schminkprodukt in der dekorativen Kosmetik dienen.

Vorzugsweise soll der Gehalt des erfindungsgemäßen kosmetischen Mittels an ionenbindenden und ionenkomplexierenden alpha-Hydroxyfettsäuren oder deren Salzen 0,01 bis 10 Gewichts-%, bezogen auf die Gesamt-Zusammensetzung des Mittels, betragen, insbesondere aber 0,1 bis 1 Gewichtsprozent, besonders bevorzugt aber 0,25 bis 0,75 Gewichtsprozent.

Als zweckmäßig hat es sich erwiesen, den pH-Wert der kosmetischen Formulierung im Bereich von 4,5 bis 9 einzustellen, insbesondere aber im Bereich von pH 5 bis pH 7.

Gegenstand der Erfindung ist auch die Verwendung eines Wirkstoffes oder mehrerer Wirkstoffe kosmetischen Zubereitungen, insbesondere kosmetischen Mitteln auf Basis üblicher kosmetischer Trägerstoffe, wobei der Wirkstoff eine erfindungsgemäße ionenbindende oder ionenkomplexierende alpha-Hydroxyfettsäure ist.

Geeignet sind beispielsweise die bekannten kosmetischen Zubereitungen wie O/W-Emulsionen, W/O-Emulsionen, Cremes, Lotionen, Milchen, Salben, Fettsalben, Gele, Tinkturen, Solubilisate, Lösungen, Suspensionen, ölige Zubereitungen oder feste, beispielsweise stiftförmige Zubereitungen, Puder oder Sprays in allen Formen.

Die Kosmetika können in bekannter Weise Trägerstoffe, Hilfsstoffe und Zusatzstoffe enthalten. Es sind z.B. wasserbindende Stoffe, Verdicker, Füllstoffe, Parfüm, Farbstoffe, Tenside, Lichtschutzmittel, Öle, Fette, Wachse, Sonnenschutzmittel, UVA-Blocker, UVB-Blocker, Vitamine, Proteine, Insektenrepellentien, Stabilisatoren, Antioxidantien, Konservierungsmittel, Alkohole, keratolytisch oder proteolytisch wirksame Substanzen, Wasser und/oder Salze, beispielsweise Puffersalze oder Puffergemische zur pH-Einstellung.

Bevorzugte erfindungsgemäße Kosmetika sind solche, die ein Lichtschutzmittel enthalten und Sonnenschutzpräparate, z.B. mit UVA- und UVB-Filtern. Solche Kosmetika neigen zu Verfärbungen, die sich erfindungsgemäß verhindern lassen.

Unerwartet zeigen die erfindungsgemäßen alpha-Hydroxyfettsäuren ein so hohes Ionenbindungsvermögen und Ionenkomplexierungsvermögen, daß sie EDTA in Kosmetika zu ersetzen vermögen. Außerdem wirkt der sich bildende Komplex in Kosmetika überraschenderweise nicht nachteilig in diesen Mitteln.

Die erfindungsgemäßen alpha-Hydroxyfettsäuren bewirken, daß keine Verfärbungen in Kosmetika auftreten, die durch Schwermetallionen hervorgerufen werden. Weiterhin besteht eine synergistische Wirkung zwischen antimikrobiell wirkenden Substanzen und den erfindungsgemäßen alpha-Hydroxyfettsäuren. Auch findet keine Oxydation von Farb- und Riechstoffen, die durch Schwermetallionen katalysiert wird, statt.
Ein besonderer Vorteil der erfindungsgemäßen alpha-Hydroxyfettsäuren hinsichtlich der Eigenschaften von z.B. EDTA zeigt sich in der guten Verträglichkeit und der guten biologischen Abbaubarkeit z.B. in Kläranlagen, Vorflutern und Gewässern, so daß es nicht zu einer Anreicherung dieser Stoffe in der Natur kommen kann.

Die Erfindung wird in den Beispielen erläutert. Auch die Präparate besitzen eine gute Wirkung und Verträglichkeit.

Zur Parfümierung sind insbesondere solche Substanzen und Parfümöle geeignet, die stabil sind und die Haut nicht reizen.

Die Erfindung ist aber nicht auf die genannten Zubereitungen und Mittel, Hilfsstoffe und Trägersubstanzen beschränkt.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden die Fettphase und die Wasserphase z.B. separat gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Die erfindungsgemäßen alpha-Hydroxy-Fettsäuren oder deren Salze können als solche oder in gelöster Form oder als Suspension (z.B. als wäßrige, alkoholische oder alkoholisch-wäßrige Lösung oder Suspension) den übrigen Bestandteilen der Formulierungen zugesetzt werden.

Gegenstand der Erfindung ist auch die Verwendung von aus Wollwachs gewonnenen α-Hydroxyfettsäuren oder aus Wollwachssäuregemischen durch Isolierungsverfahren und/oder Anreicherungsverfahren gewonnenen alpha-Hydroxyfettsäuren und alpha-Hydroxyfettsäure-Gemischen als desodorierende Wirkstoffe oder als Konservierungshelfer oder zum Binden oder Komplexieren von Ionen in kosmetischen Zubereitungen. Bevorzugt sind die hier genannten Isolierungsverfahren und Anreicherungsverfahren.

Alle Prozentangaben oder Teile beziehen sich, soweit nicht anders angegeben, auf das Gewicht, insbesondere auf das Gesamtgewicht der Zubereitungen.

Die folgenden Beispiele dienen dazu, die Erfindungen zu beschreiben, ohne daß beabsichtigt ist, die Erfindungen auf diese Beispiele zu beschränken.

### Beispiel 1

1 kg destillierte Wollwachssäure wird bei Raumtemperatur in 8 l Methanol zwei Stunden gerührt. Der nach Filtration anfallende Rückstand (200 g) enthält langkettige, vorwiegend nicht hydroxylierte Fettsäuren mit einer C-Zahl von 18 - 32.

Die bei der Filtration anfallende Lösung wird eingeengt und ergibt einen Rückstand von 800 g, der mit 5 l Cyclohexan versetzt und bei Raumtemperatur 10 Stunden gerührt wird. Die sich dabei bildende Lösung wird vom Rückstand durch erneute Filtration getrennt. Dieser Rückstand (a) wird zweimal mit je 1 l Cyclohexan gewaschen und danach getrocknet.

Die Lösung wird eingeengt und ergibt 600 g eines wachsartigen Rückstandes, der vorwiegend aus nicht hydroxylierten, kurzkettigen höheren Fettsäuren mit einer mittleren C-Zahl von 14 - 16 besteht.

Der Rückstand (a) (200 g) wird in 0,7 Liter 10%iger methanolischer Kaliumhydroxidlösung bei 40°C gelöst.

Man filtriert und säuert mit halbkonzentrierter wässriger Salzsäure auf pH3 an.

Die Extraktion mit Essigsäureethylester (dreimal mit jeweils 200 ml), Trocknen der organischen Lösung mit Natriumsulfat und Einengen ergeben 140 g eines Rückstandes, der zu ca. (Gewichtsprozent):
1. 83% aus 2-Hydroxyhexadecansäure,
2. 6% aus 2-Hydroxy-16-methylheptadecansäure,
3. 2% aus 2-Hydroxyoctadecansäure,
4. 2% aus 2-Hydroxytetradecansäure,
5. 1% aus 2-Hydroxypentadecansäure,
6. 1% aus 2-Hydroxyheptadecansäure und
7. 5% aus weiteren Homologen von 1 besteht.

### Beispiel 2

1 kg destillierte Wollwachssäure wird bei Raumtemperatur in 6 l Ethanol 1,5 Stunden gerührt. Der nach Filtration anfallende Rückstand (100 g) enthält langkettige vorwiegend nicht hydroxylierte Fettsäuren mit einer C-Zahl von 18 - 32.

Die bei der Filtration anfallende Lösung wird eingeengt und ergibt 900 g Rückstand, der mit 6 l Heptan versetzt und bei Raumtemperatur 14 Stunden gerührt wird. Die sich dabei bildende Lösung wird vom Rückstand durch erneute Filtration getrennt. Dieser Rückstand (a) wird zweimal mit je 2 l Heptan gewaschen und danach getrocknet.

Die Lösung wird eingeengt und ergibt 680 g eines wachsartigen Rückstandes, der vorwiegend aus nicht hydroxylierten kurzkettigen Fettsäuren mit einer mittleren C-Zahl von 14 - 16 besteht.

Der Rückstand (a) (220 g) wird in 0,7 Liter 10%iger methanolischer Kaliumhydroxidlösung bei 40°C gelöst. Man filtriert und säuert mit halbkonzentrierter wäßriger Salzsäure auf pH 3 an.

Die Extraktion mit Essigsäureethylester (dreimal mit jeweils 200 ml), Trocknen der organischen Lösung mit Natriumsulfat und Einengen ergeben 130 g eines Rückstandes der zu ca. (Gewichtsprozent):
1. 78% aus 2-Hydroxyhexadecansäure,
2. 8% aus 2-Hydroxy-16-methylheptadecansäure,
3. 3% aus 2-Hydroxyoctadecansäure,
4. 2% aus 2-Hydroxytetradecansäure,
5. 1% aus 2-Hydroxypentadecansäure,
6. 1% aus 2-Hydroxyheptadecansäure und
7. 7% aus weiteren Homologen von 1 besteht.

### Beispiel 3

1 kg destillierte Wollwachssäure wird bei Raumtemperatur in 7 l Cyclohexan 12 Stunden gerührt. Der sich dabei bildende Rückstand (a) wird von der Lösung durch Filtration getrennt. Die Lösung wird im Vakuum eingeengt. Man erhält 300 g eines weiteren Rückstandes, der aus langkettigen, vorwiegend nicht hydroxylierten Fettsäuren mit einer mittleren C-Zahl von 24 besteht.

Der Rückstand (a) wird mit 0,5 l Cyclohexan gewaschen. Nach Trocknung erhält man 700 g eines wachsartigen Produktes, welches in 1 l Methanol eine Stunde bei Raumtemperatur gerührt wird. Die hiervon resultierende Lösung wird vom Rückstand (b) durch Filtration getrennt und eingeengt. Man erhält dadurch 105 g einer aus 2-Hydroxyfettsäuren bestehenden Mischung folgender Zusammensetzung (Gewichtsprozent):
1. ca. 75% 2-Hydroxy-hexadecansäure,
2. ca. 5% 2-Hydroxy-16-methylheptadecansäure,
3. ca. 1% 2-Hydroxy-octadecansäure,
4. ca. 1% 2-Hydroxy-tetradecansäure,
5. ca. 8% weitere Homologe von 1. und
6. ca. 10% kurzkettige nicht hydroxylierte Fettsäuren.

Der erhaltene Rückstand (b) ergibt 595 g einer Mischung, die zu mehr als 80% aus vorwiegend nicht hydroxylierten Fettsäuren mit einer mittleren C-Zahl von 14 - 16 besteht. Außerdem finden sich in dieser Fraktion wechselnde Anteile Cholesterin und längerkettige 1,2-Diole.

### Beispiel 4

1 kg destillierte Wollwachssäure wird bei Raumtemperatur in 9 l Heptan 12 Stunden gerührt. Der sich dabei bildende Rückstand (a) wird von der Lösung durch Filtration getrennt. Die Lösung wird im Vakuum eingeengt. Man erhält 320 g eines Rückstandes, der aus langkettigen, vorwiegend nicht hydroxylierten Fettsäuren mit einer mittleren C-Zahl von 24 besteht.

Der Rückstand (a) wird mit Heptan gewaschen. Nach Trocknung erhält man 680 g eines wachsartigen Produktes, welches in 1,5 l Ethanol eine Stunde bei Raumtemperatur gerührt wird. Die resultierende Lösung wird vom Rückstand (b) durch Filtration getrennt und eingeengt. Man erhält dadurch 115 g einer aus 2-Hydroxyfettsäuren bestehenden Mischung folgender Zusammensetzung(Gewichtsprozent):
1. ca. 78% 2-Hydroxy-hexadecansäure,
2. ca. 6% 2-Hydroxy-16-methylheptadecansäure,
3. ca. 2% 2-Hydroxy-octadecansäure,
4. ca. 1% 2-Hydroxy-tetradecansäure,
5. ca. 5% weitere Homologe von 1. und
6. ca. 8% kurzkettige nicht hydroxylierte Fettsäuren

Der erhaltene Rückstand (b) ergibt 565 g einer Mischung, die zu mehr als 80% aus vorwiegend nicht hydroxylierten Fettsäuren mit einer mittleren C-Zahl von 14 - 16 besteht. Außerdem finden sich in dieser Fraktion wechselnde Anteile Cholesterin und längerkettige 1,2-Diole.

### Beispiel 5

| Pumpspray-Deo | |
|---|---|
| alpha-Hydroxyfettsäuregemisch des Beispiels 1 | 4,5 g |
| Ethanol pharm. (96%) | 35 g |
| (Parfüm, Farbstoff | nach Belieben) |
| Wasser | auf 1000 g |

Zur Herstellung der Produkte der Beispiele 5 und 6 wurden die Wirkstoffe und die Zusatzstoffe in dem Wasser-Ethanol-Gemisch gelöst.

### Beispiel 6

| Pumpspray-Deo | Gew.-Teile |
|---|---|
| alpha-Hydroxy-palmitinsäure (Racemat) | 10 |
| Ethanol pharm. (96%) | 354 |
| (Parfüm, Farbstoff | nach Belieben) |
| Wasser | auf 1000 |

### Beispiel 7

| Deo Roller (Roll-on) | |
|---|---|
| alpha-Hydroxyfettsäuregemisch mit 50 Gew.-% alpha-Hydroxy-palmitinsäure aus Wollwachssäuregemisch analog Beispiel 1 | 20 g |
| Hydroxyethylcellulose | 5 g |
| Propylenglycol | 5 g |
| Ethanol pharm. (96%) | 350 g |
| Wasser | auf 1000 g |

Hydroxyethylcellulose und Propylenglycol werden vorsuspendiert. Dann wird das Wasser zugegeben und die Masse zum Quellen stehengelassen. Das alpha-Hydroxyfettsäuregemisch wird in dem Ethanol gelöst und die Lösung dann unter Rühren in die Cellulosegelmasse gegeben.

### Beispiel 8

| Deo-Roller (Roll-on) | |
|---|---|
| alpha-Hydroxy-palmitinsäure (D) | 30 g |
| Hydroxyethylcellulose | 5 g |
| Propylenglycol | 5 g |
| Ethanol pharm. (96%) | 350 g |
| Wasser | auf 1000 g |

Es wird wie bei Beispiel 7 angegeben verfahren.

### Beispiel 9

| Desodorierendes Spray | |
|---|---|
| alpha-Hydroxy-myristinsäure (D) | 6 g |
| Ethanol pharm. (96%) | 150 g |
| Propylenglycol | 50 g |
| Dimethylether | 300 g |
| Wasser | auf 1000 g |

Der Wirkstoff wird in dem Wasser-Alkohol-Gemisch gelöst. Mit der Wirkstofflösung wird dann mit dem Treibgas Dimethylether eine Aerosol-Druckgas-Packung hergestellt.

### Beispiel 10

| Desodorierendes Spray | |
|---|---|
| alpha-Hydroxy-stearinsäure | 2,5 g |
| Ethanol pharm. (96%) | 400 g |
| 2-Octyldodecanol | 3 g |
| (Parfüm | nach Belieben) |
| Dimethylether | auf 1 000 g |

Zur Herstellung wird entsprechend Beispiel 3 verfahren.

### Beispiel 11

| Puder (desodorierend) | |
|---|---|
| alpha-Hydroxyfettsäuregemisch des Beispiels 2 | 100 g |
| Cetylstearylalkohol | 20 g |
| 2-Octyldodecanol | 20 g |
| Kaolin | 200 g |
| Talkum | 200 g |
| Aerosil | 48 g |
| Reisstärke | auf 1 000 g |

Das alpha-Hydroxyfettsäuregemisch wird bei 60°C im Octyldodecanol gelöst und in eine Mischung der übrigen Bestandteile eingerührt. Die Masse wird dann zu dem gewünschten Puder aufgearbeitet.

### Beispiel 12

| Desodorierendes Waschgelkonzentrat | |
|---|---|
| alpha-Hydroxyfettsäuregemisch des Beispiels 1 | 100 g |
| Cocoamidopropylbetain | 613 g |
| Tipa-Laurylethersulfat | 306 g |
| Citronensäure | 1 g |
| Glycerin | 10 g |
| Wasser | auf 1 000 g |

Die Komponenten werden nacheinander unter Rühren in dem Wasser gelöst.

### Beispiel 13

| Desodorierendes Shampoo | |
|---|---|
| Na-Laurethsulfate | 275 g |
| Cocoamidopropyl Betaine | 64 g |
| Kochsalz | 18 g |
| alpha-Hydroxy-palmitinsäure (D) | 10 g |
| Benzophenone-4 | 0,3 g |
| Parfüm | 12 g |
| Wasser | auf 1000 g |

Die Herstellung erfolgt wie bei Beispiel 12 angegeben.

### Beispiel 14

| Deodorant Roll on | |
|---|---|
| Methylcellulose (Viskkontran ^{R} HEC 30000) | 8 g |
| Ethoxyliertes Glycerinmonococoat 7 EO | 10 g |
| Hydriertes Rizinusöl 40 EO | 25 g |
| Ethanol | 392 g |
| Propylenglycol | 30 g |
| 2-Hydroxy-16-methylheptadecansäure (D) | 1,5 g |
| Wasser, demineralisiert | auf 1000 g |
| (EO = Ethylenoxideinheiten) | |

Die Herstellung erfolgt analog Beispiel 7, mit dem Unterschied, daß zusätzlich das Glycerinmonococoat und das Rizinusöl zu dem Propylenglycol gegeben werden.

### Beispiel 15

| Desodorierendes Pumpspray (nicht Aerosol) | |
|---|---|
| Ethanol | 615 g |
| Ethoxyliertes Glycerinmonococoat 7 EO (Cetiol^{R}HE) | 15 g |
| 2-Hydroxy-palmitinsäure | 4 g |
| Citronensäure | 0,2 g |
| Wasser, demineralisiert | auf 1000 g |

Die Komponenten werden unter Rühren in dem Wasser-Alkohol-Gemisch gelöst.

### Beispiel 16

| Desodorierendes Körperspray (Aerosol) | |
|---|---|
| Ethanol | 215 g |
| 1,2-Propylenglykol | 30 g |
| Octyldodecanol (Eutanol^{R}G) | 0,4 g |
| Parfüm | 5 g |
| alpha-Hydroxyfettsäuregemisch des Beispiels 1 | 1 g |
| Isopropylmyristat | 0,1 g |
| Treibgas | auf 1000 g |

Die Herstellung erfolgt analog Beispiel 9 mit der Lösung aller Bestandteile.

In den folgenden Beispielen bedeutet HFM: Hydroxyfettsäuregemisch des Beispiels 1 oder alpha-Hydroxy-palmitinsäure (D), die jeweils separat eingesetzt werden.

In den Beispielen 17 - 28 wirken die erfindungsgemäßen alpha-Hydroxyfettsäuren als Konservierungshelfer und ionenbindend und ionenkomplexierend.

### Beispiel 17

| O/W-Zubereitung (dickflüssig) | |
|---|---|
| Polyethylenglykol (20) oleylether (Cremophor^{R}O) | 20 g |
| Cetylstearylalkohol | 30 g |
| Paraffinöl | 50 g |
| 1,3-Propylenglykol | 30 g |
| Polyvinylpyrrolidon (Luviskol^{R}K30) | 5 g |
| HFM | 1,5 g |
| Wasser | 859 g |
| Parfüm | 4,5 g |

Die Fettphase und die Wasserphase werden auf 75°C - 85°C erwärmt, dann unter Rühren zusammengegeben und emulgiert.

### Beispiel 18

| O/W-Zubereitung (dünnflüssig) | |
|---|---|
| Ethoxylierter Fettalkohol 6 EO (Cremophor ^{R}A) | 10 g |
| Polyethylenglykol (20) oleylether (Cremophor^{R}O) | 10 g |
| Glycerinmonostearat | 20 g |
| Cetylalkohol | 10 g |
| Isopropylmyristat | 20 g |
| Glycerin | 10 g |
| Polyvinylpyrrolidon (Luviskol^{R}K30) | 5 g |
| HFM | 1, 5 g |
| Wasser | 909 g |
| Parfüm | 4,5 g |

Herstellung analog Beispiel 17

### Beispiel 19

| W/O-Creme | |
|---|---|
| Glyceryl Sorbitan Oleostearat (Arlacel 481) | 60 g |
| Ceresin (Lunacera M) | 10 g |
| Caprylic/Capric/Linoleic/Triglyceride (Miglyol 818) | 30 g |
| Mineralöl 3E | 190 g |
| Magnesium Stearate | 10 g |
| Propylenglykol | 37 g |
| HFM | 7 g |
| Wasser | 656 g |

Herstellung analog Beispiel 17

### Beispiel 20

| W/O-Lotion | |
|---|---|
| Glyceryl Sorbitan Oleostearate (Arlacel 481) | 13 g |
| PEG-7-hydrogenated Castoroil (Rizinusöl) (Arlacel 989) | 37 g |
| Caprylic/Capric/Triglyceride (Miglyol 812) | 60 g |
| Mineralöl 3E | 140 g |
| Propylenglykol | 38 g |
| HFM | 7 g |
| Wasser | 705 g |

Herstellung analog Beispiel 17

### Beispiel 21

| W/O-Emulsion | |
|---|---|
| Polyoxyäthylen-Glycerin-Sorbitan-Fettsäureester (Arlacel 988) | 36 g |
| PEG-7-hydrogenated Castor Oil (Arlacel 989) | 14 g |
| Cetearyl Alcohol (Lanette O) | 20 g |
| Mineralöl | 25 g |
| HFM | 2 g |
| Wasser | 678 g |

Herstellung analog Beispiel 17

### Beispiel 22

| Duschbad | |
|---|---|
| Sodium Laureth Sulfate | 330 g |
| Coco-ampho-di-acetat (30%) | 50 g |
| Potassium Cocoyl Hydrolysed Collagen (30%) | 110 g |
| PEG-7-Glyceryl Cocoate | 20 g |
| Cocamide MEA | 10 g |
| Sodium chloride | 5 g |
| Citric Acid (Parfüm) | 0,2 g |
| alpha-Hydroxypalmitinsäure | 5 g |
| Wasser, demineralisiert | auf 1000 g |

Die alpha-Hydroxypalmitinsäure wird im PEG-7-Glyceryl Cocoate auf 75°C erhitzt und mit der Wasser-Tensidphase vermischt.

### Beispiel 23

| Shampoo | |
|---|---|
| Sodium-Laurethsulfate | 275 g |
| Cocoamidopropyl Betaine | 64 g |
| Kochsalz | 18 g |
| alpha-Hydroxysäuregemisch des Beispiels 1 | 7,5 g |
| Benzophenone-4 | 0,3 g |
| Parfüm | 12 g |
| Wasser | auf 1000 g |

Herstellung analog Beispiel 12

Geprüft wurden die Testorganismen Escherichia coli, Pseudomonas aeruginosa, Staphylococcus epidermidis und Staphylococcus aureus. Die kosmetischen Formulierungen der Beispiele 22 und 23 wurden mit 1 - 5 x 10⁵ KBE/g angeimpft. Nach 48h waren keine Bakterien mehr nachweisbar. Dies zeigt die hervorragende Wirkung als Konservierungshelfer.

### Beispiel 24

| O/W-Emulsion | |
|---|---|
| Butylmethoxydibenzoylmethan (1-(4-t-Butylphenyl)-3-(4-methoxyphenyl)-propan-1,3-dion/Parsol^{R} 1789) | 2 g |
| alpha-Hydroxy-palmitinsäure | 5 g |
| Steareth-2 (Brij 72) | 30 g |
| Steareth-21 (Brij 721) | 20 g |
| Cetearyl Alcohol (Lanette O) | 25 g |
| Paraffin Öl | 100 g |
| Propylene Glycol | 35 g |
| FeCl₃ | 0,1 g |
| Wasser, demineralisiert | auf 1000 g |

Die Fettphase und die Wasserphase werden auf 75° - 85°C erwärmt, zusammengegeben und emulgiert.

### Beispiel 25

| W/O-Emulsion | |
|---|---|
| Butylmethoxydibenzoylmethan (1-(4-t-Butylphenyl)-3-(4-methoxyphenyl)-propan-1,3-dion/Parsol^{R} 1789) | 2 g |
| alpha-Hydroxy-palmitinsäure | 5 g |
| PEG-1 Glyceryl Oleostearate + Paraffin Wax (Arlacel 581) | 40 g |
| PEG-1 Glyceryl Sorbitan Isostearate + Paraffin Wax (Arlacel 582) | 40 g |
| Paraffin Öl | 70 g |
| Vaseline | 28 g |
| Ceresin | 22 g |
| Octyldodecanol | 100 g |
| Glycerin | 20 g |
| FeCl₃ | 0,1 g |
| Wasser, demineralisiert | auf 1000 g |

Die Fettphase und die Wasserphase werden auf 75°- 85° C erwärmt, zusammengegeben und emulgiert.

Die vorstehenden beiden Emulsionen zeigten keine Verfärbungen. Wird keine alpha-Hydroxy-palmitinsäure zugegeben, tritt eine starke Rotfärbung auf, die durch eine Reaktion des Lichtschutzmittels (Butylmethoxydibenzoylmethan) mit Eisenionen entsteht. Bisher wurden solche Verfärbungen durch den Zusatz von EDTA unterdrückt. Damit ist die hervorragende Wirkung als Bindemittel oder Komplexierungsmittel gezeigt.

### Beispiel 26

| Desodorierender Stift | |
|---|---|
| alpha-Hydroxy-palmitinsäure (D) | 7,5 g |
| Natriumstearat | 62,5 g |
| Natriumhydroxid | 12 g |
| Propylenglykol | 350 g |
| Glycerin | 500 g |
| Antioxydans (2,6-Di-tert.-butyl-4-methylphenol) | 1 g |
| Parfüm | 15 g |
| Wasser, entmineralisiert | 52 g |

Natriumstearat, Glycerin, Propylenglykol, Wasser und alpha-Hydroxypalmitinsäure werden bei 70°C aufgeschmolzen und mit Natriumhydroxid auf pH 10 eingestellt. Dann werden die übrigen Bestandteile zugegeben. Die Masse wird in Hülsen gegossen und erstarrt nach dem Abkühlen.

### Beispiel 27

| Desodorierende Seife | |
|---|---|
| 1:1 Mischung von Sodium Tallowate und Sodium Cocoate (gleiche Gewichtsteile) | 958 g |
| Vaselin | 9 g |
| Bienenwachs | 1 g |
| Titandioxide | 4 g |
| Pentanatrium-diethylentriamin-penta-acetat | 3 g |
| Kochsalz | 5 g |
| Parfüm | 10 g |
| alpha-Hydroxy-palmitinsäure | 10 g |

Die Bestandteile Sodium Tallowate und Sodium Cocoate werden zu einer Seifengrundmasse verarbeitet, in die die restlichen Komponenten eingearbeitet werden. Die Masse wird zu Seifenstücken extrudiert.

### Beispiel 28

| Duschgel | Gew.-% |
|---|---|
| Natrium-Laurylethersulfat (28 Gew.-%) | 24 |
| Cocamidopropyl-betain (30 Gew.-%) | 15 |
| PEG 7 Glycerylcocoate | 2 |
| alpha-Hydroxypalmitinsäure | 1 |
| Parfüm | 1 |
| Kochsalz | 0,5 |
| Wasser | auf 100 |

Die vorstehenden Bestandteile wurden nacheinander unter Rühren zu dem Wasser gegeben.

Mikrobiologischer Belastungstest: Zu dem vorstehenden Duschgel wurde Pseudomonas aeruginosa gegeben, so daß eine Keimzahl von 10⁶/g Duschgel vorlag.

| Ergebnis: | nach 48^{h} | nach 7 Tagen |
|---|---|---|
| | 8x10² P.aeruginosa/g | unter 10 P.aeruginosa/g |

Der entsprechende Kontrollansatz ohne alpha-Hydroxypalmitinsäure ergab dagegen keine Abtötung der Keime.

Daraus ist ersichtlich, daß der Zusatz von alpha-Hydroxyfettsäuren wie alpha-Hydroxypalmitinsäure die vollständige Abtötung der Keime bewirkt.

## Patentansprüche

1. Verwendung von alpha-Hydroxyfettsäuren mit 12 bis 24 Kohlenstoffatomen oder deren Salzen als desodorierende Wirkstoffe in kosmetischen Zubereitungen, wobei die alpha-Hydroxyfettsäuren sowohl einzeln als auch im Gemisch vorliegen können.

2. Verwendung gemäß Anspruch 1, dadurch gekennzeichnet, daß die alpha-Hydroxyfettsäuren 16 bis 18 Kohlenstoffatome besitzen.

3. Verwendung gemäß Anspruch 1, dadurch gekennzeichnet, daß die alpha-Hydroxyfettsäuren in der optisch aktiven D- oder L-Form vorliegen.

4. Verwendung gemäß Anspruch 1, dadurch gekennzeichnet, daß man alpha-Hydroxypalmitinsaure verwendet.

5. Verwendung gemäß Anspruch 1, dadurch gekennzeichnet, daß die Zubereitungen 0,01 bis 10 Gewichtsprozent, bezogen auf die Gesamt-Zubereitung, an alpha-Hydroxyfettsäure enthalten.

6. Verwendung gemäß Anspruch 1, dadurch gekennzeichnet, daß die Zubereitungen einen pH-Wert von 5 bis 7,5 besitzen.

7. Verwendung nach einem der Ansprüche 1 - 6, dadurch gekennzeichnet, daß die Zubereitungen oder Desodorantien in Form von einer W/O- oder O/W-Emulsion, Deo-Sprays, Deo-Roll-ons, Deo-Pumpsprays, desodorierenden Tinkturen, desodorierenden Intimreinigungsmittel, desodorierenden Shampoos, desodorierenden Dusch- oder Badezubereitungen, desodorierenden Pudern, desodorierenden Pudersprays, Deo-Seife oder Deo-Stiften vorliegen.

8. Verwendung von Zusammensetzungen mit einem wirksamen Gehalt von alpha-Hydroxyfettsäuren nach einem der Ansprüche 1 - 7 als kosmetische Desodorantien.

9. Verwendung von alpha-Hydroxyfettsäuren mit 10 bis 24 Kohlenstoffatomen oder deren Salzen als Konservierungshelfer in kosmetischen Zubereitungen, wobei die alpha-Hydroxyfettsäuren sowohl einzeln als auch im Gemisch vorliegen können.

10. Verwendung gemäß Anspruch 9, dadurch gekennzeichnet, daß die Zubereitungen kosmetische Rohstoffe, kosmetische Vorprodukte oder kosmetische Mittel sind.

11. Verwendung gemäß Anspruch 9, dadurch gekennzeichnet, daß die alpha-Hydroxyfettsäuren 12 bis 18 Kohlenstoffatome besitzen.

12. Verwendung gemäß Anspruch 9, dadurch gekennzeichnet, daß die alpha-Hydroxyfettsäuren in der optisch aktiven D- oder L-Form vorliegen.

13. Verwendung gemäß Anspruch 9, dadurch gekennzeichnet, daß man alpha-Hydroxypalmitinsaure verwendet.

14. Verwendung gemäß Anspruch 9, dadurch gekennzeichnet, daß man 0,01 bis 10 Gewichtsprozent, bezogen auf die Gesamt-Zubereitung, an alpha-Hydroxyfettsäure verwendet.

15. Verwendung gemäß Anspruch 9, dadurch gekennzeichnet, daß die Zubereitungen einen pH-Wert von 4,5 bis 9 besitzen.

16. Verwendung nach einem der Ansprüche 9 - 15, dadurch gekennzeichnet, daß die Zubereitungen in Form eines WAS-Produktes oder in Form von Gelen, Lösungen, Suspensionen oder Dispersionen vorliegen.

17. Verwendung von alpha-Hydroxyfettsäuren mit 12 bis 24 Kohlenstoffatomen oder deren Salzen zum Binden oder Komplexieren von Erdalkaliionen und Schwermetallionen in kosmetischen Zubereitungen, wobei die alpha-Hydroxyfettsäuren sowohl einzeln als auch im Gemisch vorliegen können.

18. Verwendung gemäß Anspruch 17, dadurch gekennzeichnet, daß die alpha-Hydroxyfettsäuren 14 bis 18 Kohlenstoffatome enthalten.

19. Verwendung gemäß Anspruch 17, dadurch gekennzeichnet, daß man alpha-Hydroxypalmitinsaure verwendet.

20. Verwendung gemäß Anspruch 17, dadurch gekennzeichnet, daß man 0,01 bis 10 Gewichtsprozent, bezogen auf die Gesamt-Zubereitung, an alpha-Hydroxyfettsäuren verwendet.

21. Verwendung gemäß Anspruch 17, dadurch gekennzeichnet, daß die Zubereitungen einen pH-Wert von 4,5 bis 9 besitzen.

22. Verwendung nach einem der Ansprüche 17 - 21, dadurch gekennzeichnet, daß die Zubereitungen Lichtschutzmittel, UVA- oder UVB-Filter enthalten oder als Sonnenschutzpräparate vorliegen.

23. Verwendung von alpha-Hydroxyfettsäuren gemäß den Ansprüchen 1, 9 und 17, dadurch gekennzeichnet, daß die alpha-Hydroxyfettsäuren aus Wollwachs oder aus Wollwachssäuregemischen durch Isolierungsverfahren und/oder Anreicherungsverfahren erhalten wurden.

## Claims

1. Use of alpha-hydroxy fatty acids with 12 to 24 carbon atoms or the salts thereof as deodorant active substances in cosmetic formulations, it being possible for the alpha-hydroxy fatty acids to be present both singly and as mixture.

2. Use according to Claim 1, characterised in that the alpha-hydroxy fatty acids have 16 to 18 carbon atoms.

3. Use according to Claim 1, characterised in that the alpha-hydroxy fatty acids are in the optically active D or L form.

4. Use according to Claim 1, characterised in that alpha-hydroxypalmitic acid is used.

5. Use according to Claim 1, characterised in that the formulations contain 0.01 to 10 per cent by weight, based on the complete formulation, of alpha-hydroxy fatty acid.

6. Use according to Claim 1, characterised in that the formulations have a pH of 5 to 7.5.

7. Use according to any of Claims 1 - 6, characterised in that the formulations or deodorants are in the form of a W/O or O/W emulsion, deodorant sprays, deodorant roll-ons, deodorant pump sprays, deodorant tinctures, deodorant intimate cleansers, deodorant shampoos, deodorant shower or bathing formulations, deodorant dusting powders, deodorant dusting powder sprays, deodorant soap or deodorant sticks.

8. Use of compositions with an effective content of alpha-hydroxy fatty acids according to any of Claims 1 - 7 as cosmetic deodorants.

9. Use of alpha-hydroxy fatty acids with 10 to 24 carbon atoms or the salts thereof as preservative aids in cosmetic formulations, it being possible for the alpha-hydroxy fatty acids to be present both singly and as mixture.

10. Use according to Claim 9, characterised in that the formulations are cosmetic raw materials, cosmetic precursors or cosmetic compositions.

11. Use according to Claim 9, characterised in that the alpha-hydroxy fatty acids have 12 to 18 carbon atoms.

12. Use according to Claim 9, characterised in that the alpha-hydroxy fatty acids are in the optically active D or L form.

13. Use according to Claim 9, characterised in that alpha-hydroxypalmitic acid is used.

14. Use according to Claim 9, characterised in that 0.01 to 10 per cent by weight, based on the complete formulation, of alpha-hydroxy fatty acid is used.

15. Use according to Claim 9, characterised in that the formulations have a pH of 4.5 to 9.

16. Use according to any of Claims 9 - 15, characterised in that the formulations are in the form of a WAS product or in the form of gels, solutions, suspensions or dispersions.

17. Use of alpha-hydroxy fatty acids with 12 to 24 carbon atoms or the salts thereof for binding or complexing alkaline earth metal ions and heavy metal ions in cosmetic formulations, it being possible for the alpha-hydroxy fatty acids to be present both singly and as mixture.

18. Use according to Claim 17, characterised in that the alpha-hydroxy fatty acids have 14 to 18 carbon atoms.

19. Use according to Claim 17, characterised in that alpha-hydroxypalmitic acid is used.

20. Use according to Claim 17, characterised in that 0.01 to 10 per cent by weight, based on the complete formulation, of alpha-hydroxy fatty acids is used.

21. Use according to Claim 17, characterised in that the formulations have a pH of 4.5 to 9.

22. Use according to any of Claims 17 - 21, characterised in that the formulations contain light protection agents, UVA or UVB filters, or are in the form of sunscreen products.

23. Use of alpha-hydroxy fatty acids according to Claims 1, 9 and 17, characterised in that the alpha-hydroxy fatty acids have been obtained from wool wax or from wool wax acid mixtures by isolation processes and/or concentration processes.

## Revendications

1. Utilisation d'acides gras α-hydroxylés ayant de 12 à 24 atomes de carbone ou de leurs sels, en tant que substances actives déodorantes dans des préparations cosmétiques, les acides gras α-hydroxylés pouvant être présents aussi bien individuellement qu'en mélange.

2. Utilisation selon la revendication 1, caractérisée en ce que les acides gras α-hydroxylés ont de 16 à 18 atomes de carbone.

3. Utilisation selon la revendication 1, caractérisée en ce que les acides gras α-hydroxylés se trouvent sous la forme optiquement active D ou L.

4. Utilisation selon la revendication 1, caractérisée en ce que l'on utilise l'acide α-hydroxypalmitique.

5. Utilisation selon la revendication 1, caractérisée en ce que les préparations contiennent de 0,01 à 10% en poids d'acide gras α-hydroxylé, par rapport à la préparation totale.

6. Utilisation selon la revendication 1, caractérisée en ce que les préparations ont un pH de 5 à 7,5.

7. Utilisation selon l'une des revendications 1 à 6, caractérisée en ce que les préparations ou déodorants se trouvent sous forme d'une émulsion E/H ou H/E de déodorants pour aérosol, de déodorants pour applicateur à bille, de déodorants pour application au moyen d'un pulvérisateur à pompe, de teintures déodorantes, de produits déodorants pour hygiéne intime, de shampooings déodorants, de préparations déodorantes pour la douche ou le bain, de poudres déodorantes, de poudres déodorantes à pulvériser, de savons déodorants ou de bâtons déodorants.

8. Utilisation de compositions ayant une teneur efficace en acides gras α-hydroxylés selon l'une des revendications 1 à 7, en tant que déodorants cosmétiques.

9. Utilisation d'acides gras α-hydroxylés ayant de 10 à 24 atomes de carbone ou de leurs sels, en tant qu'auxiliaires de conservation dans des préparations cosmétiques, les acides gras α-hydroxylès pouvant être présents aussi bien individuellement qu'en mélange.

10. Utilisation selon la revendication 9, caractérisée en ce que les préparations sont des substances brutes cosmétiques, des précurseurs cosmétiques ou des produits cosmétiques.

11. Utilisation selon la revendication 9, caractérisée en ce que les acides gras α-hydroxylés ont de 12 à 18 atomes de carbone.

12. Utilisation selon la revendication 9, caractérisée en ce que les acides gras α-hydroxylés se trouvent sous la forme optiquement active D ou L.

13. Utilisation selon la revendication 9, caractérisée en ce que l'on utilise l'acide α-hydroxypalmitique.

14. Utilisation selon la revendication 9, caractérisée en ce que l'on utilise de 0,01 à 10% en poids d'acide gras α-hydroxylé, par rapport à la préparation totale.

15. Utilisation selon la revendication 9, caractérisée en ce que les préparations ont un pH de 4,5 à 9.

16. Utilisation selon la revendication 9 à 15, caractérisée en ce que les préparations se trouvent sous forme d'un produit contenant des substances lavantes actives ou sous forme de gelées, solutions, suspensions ou dispersions.

17. Utilisation d'acides gras α-hydroxylés ayant de 12 à 24 atomes de carbone ou de leurs sels, pour la fixation ou la complexation d'ions alcalino-terreux et d'ions de métaux lourds dans des préparations cosmétiques, les acides gras α-hydroxylés pouvant être présents aussi bien individuellement qu'en mélange.

18. Utilisation selon la revendication 17, caractérisée en ce que les acides gras α-hydroxylés contiennent de 14 à 18 atomes de carbone.

19. Utilisation selon la revendication 17, caractérisée en ce que l'on utilise l'acide α-hydroxypalmitique.

20. Utilisation selon la revendication 17, caractérisée en ce que l'on utilise de 0,01 à 10% en poids d'acides gras α-hydroxylés, par rapport à la préparation totale.

21. Utilisation selon la revendication 17, caractérisée en ce que les préparations ont un pH de 4,5 à 9.

22. Utilisation selon la revendications 17 à 21, caractérisée en ce que les préparations contiennent des agents photoprotecteurs, des filtres UVA ou UVB ou se trouvent sous forme de préparations antisolaires.

23. Utilisation d'acides gras α-hydroxylés selon les revendications 1, 9 et 17, caractérisée en ce que les acides gras α-hydroxylés ont été obtenus à partir de lanoline ou de mélanges d'acides de lanoline, par des procédés d'isolement et/ou des procédés d'enrichissement.
